# EUROPÄISCHE PATENTSCHRIFT

(11) **EP 2 956 100 B1**
(45) Veröffentlichungstag und Bekanntmachung des Hinweises auf die Patenterteilung: **16.08.2017**
(21) Anmeldenummer: 14703893.9
(22) Anmeldetag: 12.02.2014
(51) Int. Cl.: A61F 13/00, A61M 1/00, A61M 27/00

(54) **ABDOMINALWUNDAUFLAGE MIT VERBINDUNGSMITTEL**
ABDOMINAL WOUND DRESSING WITH CONNECTING MEANS
PANSEMENT ABDOMINAL COMPRENANT DES MOYENS DE LIAISON

(30) Priorität: 13.02.2013 DE 102013002521
(43) Veröffentlichungstag der Anmeldung: 23.12.2015
(73) Patentinhaber: Paul Hartmann Aktiengesellschaft, 89522 Heidenheim (DE)
(72) Erfinder: CROIZAT, Pierre, 89542 Herbrechtingen (DE); ECKSTEIN, Axel, 89522 Heidenheim (DE); WOLF, Cornelia, 89542 Herbrechtingen (DE)
(74) Vertreter: Eibl, Christian
(86) Internationale Anmeldenummer: PCT/EP2014/052748
(87) Internationale Veröffentlichungsnummer: WO 2014/124983

(56) Entgegenhaltungen:
- WO-A1-01/85248
- WO-A1-2013/012381
- WO-A1-2013/034262
- WO-A1-2013/034263

## Beschreibung

Die vorliegende Erfindung betrifft eine Vorrichtung zur Verwendung bei der Unterdrucktherapie von Wunden, insbesondere von Wunden im Abdominalbereich, mit einer ersten Verbandlage mit einer ersten und einer zweiten Seite, als Organschutzschicht, wobei die erste Seite zum Aufbringen auf freigelegte innere Organe oder auf das Omentum majus vorgesehen ist, mit einer separat von der ersten Verbandlage bereit gestellten zweiten Verbandlage mit einer ersten und einer zweiten Seite, wobei die erste Seite der zweiten Verbandlage zum Aufbringen auf die zweite Seite der ersten Verbandlage vorgesehen ist sowie ein Verbindungsmittel, durch das die zweite Verbandlage nach dem Aufbringen der ersten Lage auf freigelegte innere Organe oder auf das Omentum majus mit der ersten Verbandlage verbunden werden kann, so dass ein Verschieben der ersten Verbandlage gegenüber der zweiten Verbandlage während der Therapie weitestgehend vermieden werden kann und/oder das gleichzeitige Entfernen von erster und zweiter Verbandlage erleichtert wird.

Vorrichtungen zur Unterdrucktherapie von Wunden und Verbände als Bestandteil derartiger Vorrichtungen sind im Stand der Technik bekannt. So beschreibt beispielsweise die WO1993/009727 eine Vorrichtung zur Förderung der Wundheilung durch die Applikation von Unterdruck auf dem die Wunde aufweisenden und die Wunde umgebenden Hautbereich.

Bei der Unterdrucktherapie von Wunden kommuniziert eine Unterdruck erzeugende Einrichtung über eine Saugleitung mit der Wunde oder dem Wundraum, wobei ein luft- und unterdruckdichter Wundverband zum luft- und unterdruckdichten Verschließen der Wunde und des Wundraums vorgesehen ist, so dass ein Unterdruck im Wundraum herstellbar und Flüssigkeiten aus dem Wundraum in einen typischerweise zwischen der den Unterdruck erzeugenden Einrichtung und der Wunde angeordneten Behälter absaugbar sind.

Der Ausdruck "Unterdruck" bezeichnet dabei einen innerhalb des Wundverbandes gegenüber dem Umgebungsluftdruck (atmosphärischer Luftdruck) erniedrigten Luftdruck. Unter "innerhalb des Wundverbandes" wird der durch das luftdichte Abdeckmaterial und dem Körpergewebe im Wundbereich gebildete Zwischenraum (Wundraum) verstanden. "Unterdruck" wird häufig auch als "negativer Druck" bezeichnet. Die Druckdifferenz zwischen dem Luftdruck innerhalb des Wundverbandes und dem Umgebungsluftdruck wird im Zusammenhang mit der Erfindung in mm Hg (Millimeter-Quecksilbersäule) angegeben, da dies im Bereich der Unterdrucktherapie üblich ist. 1 mm Hg entspricht einem Torr beziehungsweise 133,322 Pa (Pascal). Im Zusammenhang mit der Erfindung wird der Unterdruck, d.h. die Druckdifferenz zwischen dem Luftdruck innerhalb des Wundverbandes und dem Umgebungsluftdruck, als positiver Zahlenwert in mm Hg angegeben.

Besonders großflächige Wunden können im Abdominalbereich entweder infolge von Verletzungen oder infolge von chirurgischen Eingriffen entstehen. Chirurgische Eingriffe im Abdominalbereich werden beispielsweise bei der operativen Behandlung akuter und lebensbedrohlicher Erkrankungen des Bauchraums vorgenommen. Im Rahmen der postoperativen Versorgung derartiger chirurgischer Eingriffe kann auch die Notwendigkeit bestehen, den offenen Abdominalbereich mittels eines temporären Wundverschlusses nur vorübergehend abzudecken. Während der Behandlung einer Wunde am offenen Abdomen kann es erforderlich sein, eine sehr große Flüssigkeitsmenge, beispielsweise bis zu 5 I innerhalb von 48 Stunden, abzuleiten. Sehr große abzuleitende Flüssigkeitsmengen können z.B. während der operativen Behandlung eines Darmverschlusses (Ileus) oder einer Bauchfellentzündung (Peritonitis) entstehen. Freigelegte innere Organe oder das Omentum majus (auch als Bauchnetz oder großes Netz bezeichnet) bilden im Falle einer Abdominalwunde einen Wundgrund, also eine innerhalb eines Wundrandes befindliche Körperoberfläche. Bei der Versorgung einer Abdominalwunde wird normalerweise eine flüssigkeitsdurchlässige Materiallage (im Folgenden auch als Organschutzschicht bezeichnet) direkt auf freigelegte innere Organe oder auf das Omentum majus aufgebracht. Die den freigelegten inneren Organen oder dem Omentum majus aufliegende flüssigkeitsdurchlässige Wundkontaktschicht dient bei der Unterdruck-Behandlung einer Abdominalwunde auch als Organschutzschicht, welche ein unerwünschtes Verkleben von Organen oder dem Omentum majus mit der Bauchdecke und/oder ein unerwünschtes Verkleben von Organen oder dem Omentum majus mit einer weiteren Verbandlage, beispielsweise mit einer Wundauflage aus einem Schaum, verhindern soll. Der Randbereich der Wundauflage wird üblicherweise in den durch Bauchdecke und innere Organe gebildeten Zwischenraum eingeführt. Auf die im Gebrauch von der Wunde abgewandte Seite der Organschutzschicht wird typischerweise mindestens eine weitere flüssigkeitsdurchlässige Schicht aufgebracht. Bei der weiteren Lage handelt es sich häufig um einen porösen Polymerschaumstoff, insbesondere aus Polyurethan. Ein derartiger Verband zur temporären Abdeckung von Wunden aus Unfällen oder chirurgischen Eingriffen, insbesondere von Abdominalwunden (im Folgenden auch als "Abdominalverband" bezeichnet), ist beispielsweise aus der WO01/85248 bekannt. Der Verband ist zur Verwendung in der Unterdrucktherapie vorgesehen. Die WO01/85248 schlägt vor, den Wundgrund mit einer mit Löchern versehenen Folie abzudecken. Auf die Folie, welche die Wundkontaktschicht darstellt, wird ein poröser Schaum aufgebracht. Auf der von der Wunde abgewandten Seite umfasst der Verband einen für Flüssigkeiten undurchlässigen Abdeckfilm mit Kleberand zum luftdichten Verschließen des Wundbereichs. Weiterhin sind Verbindungsmittel vorgesehen, welche sich durch den Abdeckfilm hindurch bis zum porösen Schaum erstrecken, um den Wundraum mit einer Unterdruckquelle verbinden zu können. Im Betrieb kann Wundexsudat aus dem Wundraum entfernt werden, indem die Flüssigkeit durch die Öffnungen der gelochten Folie zunächst zum porösen Schaum und weiterhin über den Schaum zum Verbindungsmittel gelangt, welches sich im direkten Kontakt mit dem porösen Schaum befindet.

Die Patentanmeldungen DE102010052336 und WO 2013/034262 des Anmelders der vorliegenden Patentanmeldung beschreiben gleichfalls Verbände zur Verwendung bei der Unterdrucktherapie von Wunden, insbesondere von Wunden im Abdominalbereich, umfassend eine flexible perforierte Folie als Organschutzschicht und mindestens ein auf die Folie aufgebrachtes Leitungsmittel. Eine Wundauflage zur Verwendung bei der Unterdrucktherapie abdominaler Wunden, umfassend eine flexible perforierte Folie als Wundkontaktschicht, ist auch in der Patentanmeldung WO 2013/034263 desselben Anmelders offenbart. Bei der in der WO 2013/034263 beschriebenen Wundauflage ist auf der flexiblen Folie wundabseitig mindestens eine Tasche vorhanden, welche das gleichmäßige Aufbringen und Auslegen der Wundauflage auf den Wundgrund erleichtert. Die in DE102010052336, WO 2013/034262 und WO 2013/034263 beschriebenen Verbände können eine oder mehrere flüssigkeitsdurchlässige Schichten, beispielsweise aus einem Polymerschaum, umfassen.

Die WO 2013/012381 beschreibt ein Stabilisierungselement für eine zur Unterdrucktherapie vorgesehenen Wundauflage. Das Stabilisierungselement soll eine Applikation der Wundauflage erleichtern und die Wundauflage während der Therapie, beispielsweise während einer Unterdruckbehandlung des Abdominalbereichs, an seiner vorgesehenen Position fixieren. Das Stabilisierungselement steht vorzugsweise senkrecht von der Oberfläche der Wundauflage ab und kann Kanäle zur Verteilung des Unterdrucks aufweisen.

In der Praxis hat sich gezeigt, dass bei der Unterdrucktherapie des offenen Abdomens unter Verwendung von Abdominalverbänden, welche mindestens zwei Schichten aus unterschiedlichen Materiallagen umfassen, die einzelnen Materiallagen während der Behandlung unerwünscht gegeneinander verschoben werden können. Insbesondere wurde beobachtet, dass eine zunächst gleichmäßig auf freigelegte innere Organe oder auf das Omentum majus aufgebrachte Organschutzschicht während der Unterdruckbehandlung verrutschen kann. Ein gegeneinander Verschieben der einzelnen Materiallagen oder eine Verrutschen der Organschutzschicht kann zu unerwünschten Komplikationen führen, welche den Behandlungserfolg der Unterdrucktherapie gefährden können. Insbesondere kann bei einer Verschiebung der Organschutzschicht gegenüber der weiteren Materiallage während der Behandlung die weitere Materiallage in direkten Kontakt mit den freigelegten inneren Organen oder mit dem Omentum majus treten, so dass es zu Verklebungen zwischen Körpergewebe und der weiteren Materiallage kommen kann. Beim Ablösen der weiteren Materiallage, bei der es sich beispielsweise um einen Schaum handelt, vom Wundgrund beim Verbandwechsel kann es zu Beschädigungen an den freiliegenden Geweben kommen. Beschädigungen an den freiliegenden Geweben können unter anderem zu einer Fistelbildung führen. Weiterhin ist es unerwünscht, dass der Randbereich der Organschutzschicht, welcher üblicherweise in den durch Bauchdecke und innere Organe gebildeten Zwischenraum eingeführt wird, um ein unerwünschtes Verkleben von Organen oder dem Omentum majus mit der Bauchdecke zu verhindern, während der Behandlung aus dem Zwischenraum herausgezogen wird.

Der vorliegenden Erfindung liegt daher insbesondere die Aufgabe zugrunde, derartige, während der Unterdrucktherapie des offenen Abdomens auftretende Komplikationen zuverlässig zu vermeiden. Die vorliegende Erfindung möchte hierbei generell dazu beitragen, die Unterdrucktherapie des offenen Abdomens, welche meist mit schwerwiegenden und lebensbedrohlichen Krankheitszuständen einhergeht, weiter zu verbessern und die Anwendung zu erleichtern, insbesondere beim Verbandwechsel.

Erfindungsgemäß wird hierzu eine Vorrichtung, geeignet zur Verwendung bei der Unterdrucktherapie des offenen Abdomens, gemäß Anspruch 1 vorgeschlagen. Gemäß einem weiteren Aspekt der Erfindung wird ein erstes Erzeugnis zur Anwendung in der therapeutischen Behandlung des offenen Abdomens am menschlichen oder tierischen Körper mittels Unterdruck vorgeschlagen. Das erste Erzeugnis wird hierbei also ausdrücklich im Zusammenhang mit seiner speziellen medizinischen Anwendung beziehungsweise Indikation, nämlich der Unterdruck-Behandlung des offenen Abdomens, beansprucht. Das erste Erzeugnis umfasst eine erste Verbandlage, mit einer ersten und einer zweiten Seite, als Organschutzschicht, wobei die erste Seite zum Aufbringen auf einen Wundgrund, insbesondere auf freigelegte innere Organe oder auf das Omentum majus vorgesehen ist, eine separat von der ersten Verbandlage bereit gestellte zweite Verbandlage mit einer ersten und einer zweiten Seite, wobei die erste Seite der zweiten Verbandlage zum Aufbringen auf die zweite Seite der ersten Verbandlage vorgesehen ist, sowie ein Verbindungsmittel, durch das die zweite Verbandlage nach dem Aufbringen der ersten Lage auf den Wundgrund, insbesondere auf freigelegte innere Organe oder auf das Omentum majus, mit der ersten verbunden werden kann, wobei die erste Verbandlage während der Therapie durch die zweite Verbandlage an der vom Anwender gewünschten Position im Abdominalraum festgehalten werden kann und/oder wobei die erste Verbandlage nach Beendigung der Unterdrucktherapie durch Herausnehmen der zweiten Verbandlage sicher aus dem Abdominalraum entfernbar ist.

Die Erfindung betrifft gleichfalls ein zweites Erzeugnis zur Anwendung in der therapeutischen Behandlung des offenen Abdomens am menschlichen oder tierischen Körper mittels Unterdruck. Das zweite Erzeugnis umfasst eine Organschutzschicht. Das zweite Erzeugnis wird hierbei ausdrücklich im Zusammenhang mit seiner speziellen medizinischen Anwendung, nämlich der Anwendung als Organschutzschicht bei der therapeutischen Behandlung des offenen Abdomens am menschlichen oder tierischen Körper mittels Unterdruck, beansprucht. Die Organschutzschicht weist eine erste und eine zweite Seite auf, wobei die erste Seite zum Aufbringen auf einen Wundgrund, insbesondere auf freigelegte innere Organe oder auf das Omentum majus vorgesehen ist. Die zweite Seite umfasst ein Verbindungsmittel, so dass nach dem Auflegen der Organschutzschicht auf den Wundgrund, insbesondere auf freigelegte innere Organe oder auf das Omentum majus, eine Verbindung mit einer weiteren Verbandlage herstellbar ist. Vorzugsweise umfasst die Organschutzschicht ein Fluid-durchlässiges textiles Material oder eine Fluid-durchlässige flexible Folie aus einem polymeren Material. Insbesondere umfasst die Organschutzschicht eine flexible perforierte Folie, wobei die Perforationen derart in die Folie eingebracht werden, dass die Perforationsränder von der zweiten Oberfläche der Folie abstehen, so dass auf der zweiten Oberfläche der Folie dreidimensional ausgestaltete Strukturen, insbesondere kraterförmig geformte Strukturen, vorhanden sind.

Gegenstand der Erfindung ist somit auch eine Organschutzschicht zur Anwendung in der therapeutischen Behandlung des offenen Abdomens am menschlichen oder tierischen Körper mittels Unterdruck, wobei die Organschutzschicht eine erste und eine zweite Seite umfasst, und wobei die erste Seite zum Aufbringen auf einen Wundgrund, insbesondere auf freigelegte innere Organe oder auf das Omentum majus vorgesehen ist, und wobei die zweite Seite ein Verbindungsmittel umfasst, so dass nach dem Auflegen der Organschutzschicht auf den Wundgrund, insbesondere auf freigelegte innere Organe oder auf das Omentum majus, eine Verbindung mit einer weiteren Verbandlage herstellbar ist.

Ein weiterer Aspekt im Rahmen der Erfindung bezieht sich auf ein drittes Erzeugnis zur Anwendung in der therapeutischen Behandlung des offenen Abdomens am menschlichen oder tierischen Körper mittels Unterdruck. Das dritte Erzeugnis umfasst ein Verbindungsmittel, welches zur Vermeidung von Komplikationen bei der therapeutischen Behandlung angewendet wird. Das dritte Erzeugnis wird hierbei also ausdrücklich im Zusammenhang mit seiner speziellen medizinischen Indikation, nämlich der Vermeidung von Komplikationen oder Nebenwirkungen bei der Unterdruck-Behandlung des offenen Abdomens, beansprucht. Gemäß diesem Aspekt kann das Verbindungsmittel eine erste Verbandlage, welche als Organschutzschicht dient und welche zum Aufbringen auf einen Wundgrund, insbesondere auf freigelegte innere Organe oder auf das Omentum majus vorgesehen ist, mit einer zweiten Verbandlage, welche zum Aufbringen auf die erste Verbandlage vorgesehen ist, verbinden. Bei der gemäß diesem Aspekt der Erfindung zu vermeidenden Komplikation handelt es sich um ein Verrutschen der ersten Verbandlage gegenüber der zweiten Verbandlage während der therapeutischen Behandlung.

Gegenstand der Erfindung ist somit auch ein Verbindungsmittel zur Anwendung in der therapeutischen Behandlung des offenen Abdomens am menschlichen oder tierischen Körper mittels Unterdruck, wobei das Verbindungsmittel eine erste Verbandlage, welche als Organschutzschicht dient und welche zum Aufbringen auf einen Wundgrund, insbesondere auf freigelegte innere Organe oder auf das Omentum majus vorgesehen ist, mit einer zweiten Verbandlage, welche zum Aufbringen auf die erste Verbandlage vorgesehen ist, verbinden kann, dadurch gekennzeichnet, dass das Verbindungsmittel zum Vermeiden einer während der Behandlung auftretenden Komplikation indiziert ist, wobei es sich bei der Komplikation um das Verkleben der zweiten Verbandlage mit freigelegten inneren Organen oder um das Verkleben der zweiten Verbandlage mit dem Omentum majus infolge einer Verschiebung der ersten Verbandlage gegenüber der zweiten Verbandlage während der therapeutischen Behandlung handelt.

Des Weiteren bezieht sich die Erfindung auf ein viertes Erzeugnis zur Anwendung in der therapeutischen Behandlung des offenen Abdomens am menschlichen oder tierischen Körper mittels Unterdruck. Das vierte Erzeugnis umfasst ein Verbindungsmittel, welches zur Vereinfachung der therapeutischen Behandlung des offenen Abdomens am menschlichen oder tierischen Körper mittels Unterdruck angewendet wird. Das vierte Erzeugnis wird hierbei also ausdrücklich im Zusammenhang mit einer weiteren speziellen medizinischen Indikation, nämlich der Vereinfachung der therapeutischen Behandlung des offenen Abdomens, beansprucht. Gemäß diesem Aspekt kann das Verbindungsmittel eine erste Verbandlage, welche als Organschutzschicht dient und welche zum Aufbringen auf einen Wundgrund, insbesondere auf freigelegte innere Organe oder auf das Omentum majus vorgesehen ist, mit einer zweiten Verbandlage, welche zum Aufbringen auf die erste Verbandlage vorgesehen ist, verbinden. Die beanspruchte Vereinfachung der therapeutischen Behandlung des offenen Abdomens besteht darin, dass die erste Verbandlage nach Beendigung der Unterdrucktherapie durch Herausnehmen der mit der ersten Verbandlage verbundenen zweiten Verbandlage einfach und sicher aus dem Abdominalraum entfernbar ist.

Gegenstand der Erfindung ist somit auch ein Verbindungsmittel zur Anwendung in der therapeutischen Behandlung des offenen Abdomens am menschlichen oder tierischen Körper mittels Unterdruck, wobei das Verbindungsmittel eine erste Verbandlage, welche als Organschutzschicht dient und welche zum Aufbringen auf einen Wundgrund, insbesondere auf freigelegte innere Organe oder auf das Omentum majus vorgesehen ist, mit einer zweiten Verbandlage, welche zum Aufbringen auf die erste Verbandlage vorgesehen ist, verbinden kann, dadurch gekennzeichnet, dass das Verbindungsmittel die Behandlung vereinfacht, indem die erste Verbandlage nach Beendigung der Unterdrucktherapie durch Herausnehmen der mit der ersten Verbandlage verbundenen zweiten Verbandlage einfach und sicher aus dem Abdominalraum entfernbar ist.

Die Erfindung bezieht sich gleichermaßen auf ein Verfahren zur therapeutischen Behandlung des offenen Abdomens am menschlichen oder tierischen Körper mittels Unterdruck, umfassend
- Bereitstellen einer Unterdruckquelle sowie optional eines Behälters für die abgesaugten Wundfluide,
- Bereitstellen eines geeigneten Mittels zum Herstellen einer Unterdruckkommunikation zwischen Unterdruckquelle und Wundraum, beispielsweise eine Unterdruckleitung und ein Unterdruckanschlussstück (Port),
- Bereitstellen eines geeigneten Mittels zum Versiegeln des Wundraums, beispielsweise eine luftdichte Abdeckfolie,
- Bereitstellen einer ersten Verbandlage mit einer ersten und einer zweiten Seite,
- Bereitstellen einer zweiten Verbandlage mit einer ersten und einer zweiten Seite,
- Bereitstellen eines Verbindungsmittels, durch das die zweite Verbandlage mit der ersten Verbandlage dauerhaft oder vorübergehend verbunden werden kann,
- Auflegen der ersten Seite der ersten Verbandlage auf einen Wundgrund, insbesondere auf freigelegte innere Organe oder auf das Omentum majus,
- Auflegen der ersten Seite einer zweiten Verbandlage auf die zweite Seite der ersten Verbandlage,
- Herstellen einer dauerhaften oder vorübergehenden Verbindung zwischen erster Verbandlage und zweiter Verbandlage,
- optional Aufbringen weiterer Verbandlagen,
- Herstellen einer luftdichten Abdeckung des Abdominalraumes und Anschluss einer Unterdruckquelle,
- Durchführen der Unterdrucktherapie.

Die hier vorgeschlagenen Vorrichtungen, Erzeugnisse, Verbandkomponenten und Verfahren können mit üblichen, dem Fachmann bekannten Vorrichtungen zur Unterdrucktherapie von Wunden verwendet werden. Derartige, aus dem Stand der Technik bekannte Vorrichtungen zur Unterdrucktherapie von Wunden umfassen beispielsweise eine Unterdruckquelle, einen Behälter zur Aufnahme des aus dem Wundraum abgesaugten Exsudates, ein oder mehrere Leitungsmittel, ein Unterdruckanschlussstück sowie eine luftdichte, selbstklebende Folie zum Abdichten des Wundraumes.

Die hier vorgestellte Vorrichtung umfasst wenigstens zwei Verbandlagen, wobei die Verbandlagen vor der Anwendung separat bereit gestellt werden.

Bei der ersten Verbandlage handelt es sich um eine Fluid-durchlässige Organschutzschicht, welche zum Aufbringen beziehungsweise Auflegen auf einen Wundgrund, insbesondere zum Aufbringen beziehungsweise Auflegen auf freigelegte innere Organe oder auf das Omentum majus vorgesehen ist. Der Randbereich der Organschutzschicht wird üblicherweise nach dem Auflegen des zentralen Bereichs der Organschutzschicht in den durch Bauchdecke und innere Organe gebildeten Zwischenraum eingeführt. Hierdurch soll ein unerwünschtes Verkleben von Organen oder dem Omentum majus mit der Bauchdecke verhindert werden. Die Organschutzschicht umfasst vorzugsweise ein Fluid-durchlässiges textiles Material oder eine Fluid-durchlässige flexible Folie aus einem polymeren Material, insbesondere eine flexible, perforierte Folie. Es ist wesentlich, dass die Organschutzschicht aus einem Material besteht, welches während der Dauer der Anwendung nicht mit den freigelegten inneren Organen, dem Omentum majus oder der Bauchdecke verklebt oder verwächst. Das Material soll atraumatische Eigenschaften aufweisen. Bevorzugt umfasst die Organschutzschicht eine Folie, insbesondere eine thermoplastische Folie. Geeignete Materialien für eine thermoplastische Folie umfassen insbesondere Ethylvinylacetat (EVA), Polyurethan (PU), Polyethylen (PE), Polyethylenterephthalat (PET), Polytetrafluorethylen (PTFE), Polyvinylchlorid (PVC), thermoplastische Elastomere (TPE), Polyorganosiloxan (Silikon) oder einer Mischung daraus. Unter der Bezeichung TPE sind in diesem Zusammenhang thermoplastische Elastomere auf Olefinbasis (TPO), vernetzte thermoplastische Elastomere auf Olefinbasis (TPV), thermoplastische Elastomere auf Urethanbasis (TPU), thermoplastische Polyesterelastomere bzw. thermoplastische Copolyester (TPC), Styrol-Blockcopolymere (TPS) und thermoplastische Copolyamide (TPA) umfasst. Vorzugsweise handelt es sich bei der thermoplastischen Folie um eine Polyethylen-Folie.

Das Flächengewicht der ersten Folie sollte mindestens 30 g / m² und höchstens 150 g / m², vorzugsweise mindestens 45 g / m² und höchstens 95 g / m² und insbesondere mindestens 55 g / m² und höchstens 65 g / m² betragen.

Normalerweise liegt die Organschutzschicht als einlagige Materialschicht vor. Alternativ kann jedoch auch eine mehrlagige Struktur, deren unterschiedliche Lagen unlösbar und somit laminat-artig verbunden sind, als Organschutzschicht zum Einsatz kommen. Die unterschiedlichen Lagen können aus demselben oder aus unterschiedlichen Materialien gefertigt sein, welche dann herstellerseitig zu einem Laminat verbunden werden. Auf der ersten und/oder zweiten Oberfläche der ersten Verbandlage kann eine dreidimensionale Struktur vorhanden sein, beispielsweise Perforationen mit dreidimensional ausgebildeten Rändern. Die Dicke der ein- oder mehrlagigen Organschutzschicht beträgt insgesamt, einschließlich gegebenenfalls an der Oberfläche vorhandener dreidimensionaler Strukturen, 10 bis 1000 µm, vorzugsweise 20 bis 500 µm.

Die erste Seite der zweiten Verbandlage wird nach der Applikation der ersten Verbandlage auf die zweite Seite der ersten Verbandlage aufgebracht. Die zweite Verbandlage weist im Vergleich zur ersten Verbandlage vorzugsweise eine kleinere Fläche auf, insbesondere beträgt die Fläche der ersten Seite der zweiten Verbandlage mindestens 3 % und höchstens 97 %, insbesondere mindestens 25 % und höchstens 90 % der Fläche der ersten Seite der ersten Verbandlage. Die zweite Verbandlage muss Fluid-durchlässig und Fluid-leitend ausgebildet sein, so dass einerseits aus aus der Wunde abgesaugtes Fluid zur Unterdruckquelle hin transportiert und anderseits eine gleichmäßige Verteilung des Unterdrucks im Wundraum gewährleistet werden kann. Die zweite Verbandlage dient weiterhin einer gleichmäßigen Verteilung des auf den Wundgrund während der Unterdruckbehandlung ausgeübten mechanischen Druckes. Hierzu sollte die zweite Verbandlage vorzugsweise eine im Vergleich zur ersten Verbandlage wesentlich höhere Dicke aufweisen, beispielsweise 2 mm bis 100 mm, insbesondere 5 mm bis 50 mm. In der Praxis wird die zweite Verbandlage häufig so aufgebracht, dass die zweite Verbandlage zwischen den Wundränder angeordnet ist. Dies ist insbesondere dann erforderlich, wenn ein temporärer Wundverschluss hergestellt werden soll. In diesem Falle sollte die Dicke der zweiten Verbandlage der Dicke der Bauchdecke entsprechen.

Geeignete Materialien zur Herstellung der zweiten Verbandlage umfassen textile oder polymere Materialien, insbesondere einen Schaum aus Polyurethan, einen Schaum aus Silikon oder einen Schaum aus Polyvinylalkohol (PVA).

Gemäß einer bevorzugten Ausführungsform der Erfindung umfasst die zweite Verbandlage eine oder mehrere Schichten eines porösen Polymerschaumstoffs. Besonders geeignet ist dabei ein offenzelliger Polymerschaumstoff. Im Rahmen dieser Anmeldung bedeutet der Begriff offenzellig, dass im Schaumstoff mindestens 60 % offene Zellen, bevorzugt mindestens 90 % offene Zellen, mehr bevorzugt mindestens 98 % offene Zellen, insbesondere im Wesentlichen 100 % offene Zellen, bezogen auf die Gesamtzahl an Zellen, vorhanden sind.

Geeignete Materialien für einen porösen Schaumstoff umfassen beispielsweise Polyurethan, Polyurethan-Polyharnstoff-Copolymere, Polyvinylalkohol (PVA) oder Silikon.

Alternativ oder zusätzlich kann die flüssigkeitsdurchlässige Schicht textile Materialien wie Woven oder Non-woven umfassen, beispielsweise einen Vliesstoff aus synthetischen Polymeren wie Polyamid, Polyester oder Polypropylen.

Die zweite Verbandlage kann einlagig ausgebildet sein, oder mehrere Schichten umfassen. Im Falle einer mehrere Schichten umfassenden zweiten Verbandlage ist es nicht erforderlich, dass die Schichten unlösbar miteinander verbunden sind. Die einzelnen Schichten können separat bereitgehalten werden und nacheinander appliziert werden. So ist es beispielsweise möglich, dass mehrere Lagen eines porösen Schaumstoffs nacheinander appliziert werden, bis die zweite Schaumlage eine nach Einschätzung des behandelnden Arztes ausreichende Dicke aufweist.

Erfindungsgemäß umfasst die Vorrichtung ein Verbindungsmittel, durch das die zweite Verbandlage nach dem Aufbringen der ersten Lage auf den Wundgrund, insbesondere auf freigelegte innere Organe oder auf das Omentum majus, mit der ersten Verbandlage verbunden werden kann. Unter Verbindungsmittel wird im Zusammenhang mit der vorliegenden Erfindung jedes Mittel verstanden, welches eine permanente oder vorübergehende Verbindung von erster und zweiter Verbandlage gewährleistet.

Das Verbindungsmittel kann an der ersten und/oder an der zweiten Verbandlage vorhanden bzw. ein integraler Bestandteil von erster und/oder zweiter Verbandlage sein. Alternativ kann es sich bei dem Verbindungsmittel um ein von erster und/oder zweiter Verbandlage separat vorhandenes Mittel handeln. Das Verbindungsmittel umfasst Mittel zur formschlüssigen Verbindung, Mittel zur kraftschlüssigen Verbindung und Mittel zur stoffschlüssigen Verbindung. Nicht limitierende Beispiele für im Zusammenhang mit der vorliegenden Erfindung verwendbare formschlüssige Verbindungsmittel umfassen einschnappende Mittel, einhakende Mittel, zueinander komplementäre Oberflächenstrukturen oder Reißverschlüsse. Bevorzugte formschlüssige Verbindungsmittel umfassen beispielsweise sich gegenseitig verhakende Oberflächenelemente oder separat aufgebrachte Klettelemente. Nicht limitierende Beispiele für im Zusammenhang mit der vorliegenden Erfindung verwendbare kraftschlüssige Verbindungsmittel umfassen miteinander verknotbare Materialstreifen oder Bänder sowie Klemmen. Nicht limitierende Beispiele für im Zusammenhang mit der vorliegenden Erfindung verwendbare stoffschlüssige Verbindungsmittel umfassen adhäsive oder klebende Oberflächen sowie separat aufgebrachte Klebemittel, Klebestreifen oder Klebeschichten.

Im Zusammenhang mit der vorliegenden Erfindung besonders bevorzugte Verbindungsmittel umfassen insbesondere auf der zweiten Seite der ersten Verbandlage und/oder auf der ersten Seite der zweiten Verbandlage aufgebrachte Klettelemente sowie an der ersten Verbandlage und an der zweiten Verbandlage vorhandene und miteinander verknotbare oder vernähbare Materialstreifen, Bänder oder Fäden. Weitere besonders bevorzugte Verbindungsmittel umfassen an der Oberfläche der zweiten Seite der ersten Verbandlage und an der Oberfläche der ersten Seite der zweiten Verbandlage vorhandene dreidimensional ausgestaltete Strukturen, welche eine Haftung zwischen den beiden Oberflächen bewirken.

Das Verbindungsmittel kann eine Mehrzahl einzelner Verbindungselemente umfassen. Das Verbindungsmittel ermöglicht dem Anwender, also beispielsweise dem behandelnden Arzt, eine lösbare oder unlösbare Verbindung von erster Verbandlage und zweiter Verbandlage nach dem Aufbringen der ersten Verbandlage herzustellen, so dass ein Verschieben der ersten Verbandlage gegenüber der zweiten Verbandlage während der Unterdrucktherapie weitestgehend vermieden werden kann. In der Praxis hat sich zudem gezeigt, dass eine erfindungsgemäße Vorrichtung besonders leicht aus dem Wundraum entfernt werden kann, da die zunächst separat applizierten Verbandlagen beim Verbandswechsel aneinander haften. Es ist daher nicht erforderlich, die Organschutzschicht und die weitere Lage einzeln abzunehmen. Dies erleichtert die Handhabung des Verbandes beim Verbandswechsel.

Ein Vorteil der vorliegenden Erfindung gegenüber einem herstellerseitig gefertigten Laminat aus erster und zweiter Verbandlage besteht darin, dass die lösbare oder unlösbare Verbindung von erster Verbandlage und zweiter Verbandlage erst nach dem Aufbringen der ersten Verbandlage zustande kommt. Somit können beide Verbandlagen unabhängig voneinander an die Wundverhältnisse angepasst und appliziert werden.

Grundsätzlich können im Zusammenhang mit der vorliegenden Erfindung Verbindungsmittel verwendet werden, welche nach der Applikation der zweiten Verbandlage auf die zweite Oberfläche der ersten Verbandlage eine permanente und unlösbare Verbindung von erster und zweiter Verbandlage herzustellen erlauben. In gleicher Weise können Verbindungsmittel verwendet werden, welche nach der Applikation der zweiten Verbandlage auf die zweite Oberfläche der ersten Verbandlage eine nur vorübergehende und lösbare Verbindung von erster und zweiter Verbandlage bewirken, solange für die Dauer der Unterdruck-Applikation eine ausreichende Haftung von erster und zweiter Verbandlage gewährleistet ist, so dass ein Verschieben der ersten Verbandlage gegenüber der zweiten Verbandlage während der Therapie weitestgehend vermieden werden kann. Eine ausreichende Haftung von erster und zweiter Verbandlage ist dann gewährleistet, wenn zum Verschieben der ersten Verbandlage gegen die zweite Verbandlage im nassen Zustand eine statische Gleitreibungskraft Fₛ von mindestens 3 N, vorzugsweise von mindestens 5 N erforderlich, ist und/oder wenn zum Verschieben der ersten Verbandlage gegen die zweite Verbandlage im trockenen Zustand eine statische Gleitreibungskraft Fₛ von mindestens 6 N, vorzugsweise von mindestens 9 N, erforderlich ist (jeweils gemessen nach DIN EN ISO 8295, wie im Ausführungsbeispiel näher dargestellt). Da die zwischen den Oberflächen vorhandene Gleitreibungskraft herstellungsbedingt in Längsrichtung ("Maschinenrichtung", auch als "MD" bezeichnet) und Querrichtung der Materialien variieren kann, wird hier unter einer "mindestens erforderlichen statischen Gleitreibungskraft Fₛ" die bei unterschiedlichen Orientierungen der Lagen zueinander geringste auftretende statische Gleitreibungskraft Fₛ verstanden. Hierdurch kann gewährleitet werden, dass ein Verschieben der ersten Verbandlage gegenüber der zweiten Verbandlage während der Therapie in jeder Richtung und unabhängig der Orientierung der Lagen zueinander weitestgehend vermieden werden kann.

Die erfindungsgemäße Vorrichtung umfasst eine erste Verbandlage, eine zweite Verbandlage sowie ein Verbindungsmittel wie vorstehend beschrieben, wobei zum Verschieben der ersten Verbandlage im nassen Zustand gegen die zweite Verbandlage im nassen Zustand eine statische Gleitreibungskraft Fₛ von mindestens 3 N erforderlich ist und/oder wobei zum Verschieben der ersten Verbandlage im trockenen Zustand gegen die zweite Verbandlage im trockenen Zustand eine statische Gleitreibungskraft Fₛ von mindestens 6 N erforderlich ist.

Im Rahmen der vorliegenden Erfindung sind Ausführungsformen denkbar, wonach das Verbindungsmittel insbesondere auf der zweiten Seite der ersten Verbandlage vorhanden ist. Gemäß einer besonders bevorzugten Ausführungsform umfasst die Vorrichtung deshalb eine Organschutzschicht, insbesondere eine flexible Folie als Organschutzschicht, mit einer ersten und einer zweiten Seite, zur Verwendung bei der Unterdrucktherapie des offenen Abdomens, wobei die erste Seite zum Aufbringen auf freigelegte innere Organe oder auf das Omentum majus vorgesehen ist und wobei die zweite Seite ein Verbindungsmittel umfasst, so dass nach dem Auflegen der Organschutzschicht auf freigelegte innere Organe oder auf das Omentum majus eine Verbindung mit einer weiteren Verbandlage herstellbar ist. Im Zusammenhang mit dieser bevorzugten Ausführungsform können insbesondere an der ersten Verbandlage Folienstreifen, Bänder oder Fäden vorhanden sein, welche nach der Applikation der weiteren Verbandlage mit der weiteren Verbandlage verbunden werden können. Hierzu können die Folienstreifen, Bänder oder Fäden beispielsweise mit der weiteren Verbandlage vernäht oder verknotet werden. Es ist auch denkbar, an der weiteren Verbandlage herstellerseitig Schlitze vorzusehen, durch welche die Folienstreifen, Bänder oder Fäden hindurchgeführt werden können, um die erste Verbandlage mit der weiteren Verbandlage zu verbinden. Gleichfalls wäre denkbar, dass der Anwender in die zweite Verbandlage unmittelbar vor der Applikation der zweiten Verbandlage an geeigneter Stelle Öffnungen oder Einschnitte einbringt, durch welche ein oder mehrere freie Enden der Folienstreifen, Bänder oder Fäden hindurchgeführt und gegebenenfalls verknotet werden können.

Alternativ kann die zweite Seite der ersten Verbandlage eine haftende oder adhäsive Oberfläche umfassen, so dass nach Applikation der weiteren Verbandlage eine haftende Verbindung zwischen erster Verbandlage und weiterer Verbandlage herstellbar ist. Die haftende Verbindung ist erfindungsgemäß dergestalt, dass zum Verschieben der ersten Verbandlage im trockenen Zustand gegen die zweite Verbandlage im trockenen Zustand eine statische Gleitreibungskraft Fₛ von mindestens 6 N erforderlich ist. Gemäß einer bevorzugten Ausführungsform ist es somit möglich, dass das Verbindungsmittel lediglich auf der zweiten Seite der ersten Verbandlage vorhanden ist. Die Verbindung von erster und zweiter Verbandlage wird in diesem Falle ausschließlich durch ein an der ersten Verbandlage vorhandenes Verbindungsmittel bewirkt, wobei das Verbindungsmittel vorzugsweise an der Oberfläche der zweiten Seite der ersten Verbandlage vorhanden ist.

Nach einer weiteren bevorzugten Ausführungsform, die im Rahmen der Erfindung von besonderer Bedeutung in der medizinischen Praxis ist, umfasst das Verbindungsmittel sowohl die Oberfläche der zweiten Seite der ersten Verbandlage als auch die Oberfläche der ersten Seite der zweiten Verbandlage. Hierbei ist insbesondere vorgesehen, dass die Oberfläche der zweiten Seite der ersten Verbandlage und die Oberfläche der ersten Seite der zweiten Verbandlage dreidimensional ausgestaltete Strukturen (auch als 3-D-Strukturen bezeichnet) umfassen, welche eine Haftung zwischen den beiden Oberflächen bewirken. Nach dieser Ausführungsform kann grundsätzlich jede Art von dreidimensional ausgebildeter Oberflächenstruktur in einer die Verbandlagen verbindenden Weise funktionalisiert werden, solange mittels der Strukturen eine formschlüssige oder kraftschlüssige vorübergehende oder dauerhafte Verbindung von erster und zweiter Verbandlage während der Unterdrucktherapie bewerkstelligt werden kann. Es ist hierbei nicht zwangsläufig erforderlich, dass die in verbindender Funktion zum Einsatz gebrachten Strukturen ausschließlich oder in erster Linie zur weitestgehenden Vermeidung eines Verschiebens der ersten Verbandlage gegenüber der zweiten Verbandlage und/oder zum gleichzeitigen Entfernen von erster und zweiter Verbandlage beim Verbandwechsel aufgebracht werden. Genauso wäre vorstellbar, dass die eine formschlüssige oder kraftschlüssige Verbindung bewirkenden Oberflächenstrukturen hinsichtlich weiterer, hier nicht näher ausgeführter Funktionen auf der Oberfläche vorhanden sind, soweit und solange es dem Anwender möglich ist, eine temporäre oder permanente Verbindung zwischen erster und zweiter Verbandlage herzustellen, nachdem er die zweite Verbandlage auf die erste Verbandlage aufgebracht hat.

Auf der Oberfläche der zweiten Seite der ersten Verbandlage und der Oberfläche der ersten Seite der zweiten Verbandlage vorhandene dreidimensional ausgestaltete Strukturen können integraler Bestandteil der Verbandlage sein, oder herstellerseitig als separate Komponente auf die jeweilige Oberfläche aufgebracht sein. Als nichtausschließliches Beispiel für eine herstellerseitig separat auf eine Oberfläche aufgebrachte dreidimensional ausgestaltete Struktur werden hier streifenartig, ringförmig oder punktartig unlösbar auf die Verbandlage aufgebrachte Klettelemente sowie an der Oberfläche der Verbandlage unlösbar befestigte Folienstreifen oder Bänder vorgeschlagen. Als nichtausschließliche Beispiele für eine 3-D Struktur, welche integraler Bestandteil der Verbandlage sein kann, wird hier eine dreidimensional ausgestaltete Perforation in einer Folie sowie eine dreidimensional geformte Oberfläche eines offenzelligen Schaumes vorgeschlagen. Bei der vorgenannten Lochfolie, welche im Rahmen der Erfindung insbesondere als Organschutzschicht verwendet werden kann, können die dreidimensional eingebrachten Perforationen beispielsweise kraterartig aus der Planebene der Folie herausragen, so dass eine Mikrostruktur entsteht, welche mit einem geeigneten Widerpart in der zweiten Verbandlage eine haftende Verbindung eingehen kann. Hinsichtlich des gleichfalls beispielhaft genannten offenzelligen Schaums, welcher im Rahmen der Erfindung vorzugsweise als zweite Verbandlage in Frage kommt, kann die eine Haftung ermöglichende dreidimensional ausgestaltete Mikrostruktur durch die Stege der Schaumzellen und/oder durch zur Oberfläche des Schaumes hin offene Hohlräume bereit gestellt werden. Um den erwünschten Hafteffekt zu erzielen, muss bei der Auswahl des zur Herstellung der zweiten Verbandlage verwendeten Schaummaterials darauf geachtet werden, dass ein ausreichender Anteil an Schaum-Stegen an oder nahe der Schaumoberfläche vorhanden und somit dem Zugriff haftender Mikrostrukturen der ersten Verbandlage ausgesetzt ist und/oder dass eine ausreichende Anzahl zur Oberfläche des Schaumes hin offene Hohlräume vorhanden ist. Die Stege sind im Wesentlichen innerhalb des Schaumstoffkörpers vorhanden, so dass sie üblicherweise nicht über die Ebene der dreidimensional strukturieren Schaumoberfläche herausstehen. Eine hakende oder anderweitige formschlüssige Wechselwirkung der auf der Oberfläche der ersten Verbandlage vorhandenen dreidimensionalen Strukturen mit den Mikrostrukturen auf der Oberfläche der zweiten Verbandlage, namentlich die Perforationsränder und die Stege des Schaums, kann nur stattfinden, wenn die beteiligten Strukturelemente hinsichtlich ihrer Abmessungen aufeinander abgestimmt sind. Eine haftende Interaktion mit den auf der ersten Verbandlage vorhandenen dreidimensional ausgestalteten Mikrostrukturen kann alternativ oder zusätzlich, also in einer die Haftung verstärkenden Weise, durch eine Vielzahl von zur ersten Oberfläche des Schaumes hin offener Hohlräume erreicht werden, in welche die auf der zweiten Seite der ersten Verbandlage vorhandenen Mikrostrukturen eindringen können, so dass eine form- und/oder kraftschlüssige Verbindung entsteht. Auf mikroskopischer Ebene betrachtet, weist die Oberfläche eines offenzelligen Polymerschaumes, welcher eine Vielzahl zur Oberfläche des Schaumes hin offenen Hohlräumen umfasst, eine dreidimensional ausgestalte Oberflächenstruktur auf. Bei den zur Oberfläche hin offenen Hohlräumen handelt es sich um im Inneren des Schaumstoffes vorhandene Zellen, welche durch einen bei der Herstellung der Verbandlage erforderlichen Zuschnitt des Schaumstoffes teilweise freigelegt werden. Um eine haftende Interaktion zwischen den an der ersten Verbandlage vorhandenen Mikrostrukturen und den an der zweiten Verbandlage vorhandenen Hohlräumen zu erzielen, ist es gleichfalls erforderlich, dass die an beiden Verbandlagen vorhandenen Mikrostrukturen und die zur Oberfläche des Schaums hin offenen Hohlräume hinsichtlich ihrer Abmessungen aufeinander abgestimmt sind. Eine Zelle ist der bei der Herstellung von Schaumstoffen gebildete einzelne Hohlraum, der von Zellwänden und/oder Zellstegen teilweise oder vollständig umschlossen ist. Eine geschlossene Zelle ist üblicherweise eine Zelle, die vollständig von ihren Wänden umschlossen ist und daher nicht mit anderen Zellen über die Gasphase in Verbindung steht. Eine offene Zelle ist üblicherweise eine Zelle, die über die Gasphase mit anderen Zellen in Verbindung steht. Im Rahmen dieser Anmeldung bedeutet der Begriff offenzellig, dass im Schaumstoff mindestens 60 % offene Zellen, bevorzugt mindestens 90 % offene Zellen, mehr bevorzugt mindestens 98 % offene Zellen, insbesondere im Wesentlichen 100 % offene Zellen, bezogen auf die Gesamtzahl an Zellen, vorhanden sind. Die Offenzelligkeit des Schaumstoffs wird üblicherweise nach ASTM D 2856-87, Verfahren B) bestimmt. Unter Zellwand wird üblicherweise die die Zelle umschließende Wand verstanden. Die Zellwand kann auch als Zellmembran bezeichnet werden. Als Zellsteg oder Steg wird üblicherweise der Bereich der Zellwand verstanden, der mehr als zwei Zellen voneinander trennt. Bei dem im Zusammenhang mit der Erfindung als zweite Verbandlage vorzugsweise verwendbaren offenzelligen Schaumstoff kann es sich um einen retikulierten oder um einen nichtretikulierten Schaumstoff handeln. Unter einem retikulierten Schaumstoff wird ein Schaumstoff verstanden, der im Wesentlichen nur Stege aufweist. Bei einem retikulierten Schaumstoff sind die Zellwände somit im Wesentlichen entfernt.

Nach einer besonders bevorzugten Ausführungsform handelt es sich bei der ersten Verbandlage, also bei der Organschutzschicht, um eine flexible, perforierte Folie, insbesondere um eine Folie aus Polyethylen, welche dreidimensional ausgestaltete Perforationen umfasst. Wie bereits dargestellt, erweist es sich hier als besonders vorteilhaft, wenn die Perforationen derart in die Folie eingebracht werden, dass die Ränder von der zweiten Oberfläche der Folie abstehen, so dass auf der zweiten Oberfläche der Folie eine dreidimensional ausgestaltete Struktur, insbesondere eine kraterförmig geformte Struktur, vorhanden ist. Hierbei kommt es darauf an, dass die vorzugsweise kraterförmige Struktur in einem die Haftung ermöglichendem Maße über die plane Oberfläche der Folie hinausragt. Allerdings sollte die Struktur nicht allzu weit aus der Oberfläche herausragen, da ansonsten die Festigkeit der hakenden Mikrostrukturen nachlassen kann. Die Struktur weist deshalb vorzugsweise eine Höhe von mindestens 100 µm und höchstens 2000 µm, insbesondere von mindestens 200 µm und höchstens 1000 µm und ganz besonders bevorzugt von mindestens 250 µm und höchstens 600 µm auf. Unter der Höhe wird hier die senkrecht zur Ebene der Folie gemessene maximale Erstreckung der dreidimensionalen Struktur verstanden. Die Messung kann anhand geeigneter mikroskopischer Aufnahmen von Folienquerschnitten ermittelt werden. Weiterhin wird die Haftungswirkung der durch die Perforation gebildeten Mikrostrukturen vom Winkel beeinflusst, der durch die Ebene der Kraterwände gegen die Ebene der planen Folie gebildet wird. Hier hat es sich gezeigt, dass eine wirksame Haftung insbesondere dann beobachtbar ist, wenn der vorgenannte Winkel mindestens 10° und höchstens 90°, vorzugsweise mindestens 45° und höchstens 80° beträgt.
Gemäß einer weiteren besonders bevorzugten Ausführungsform, welche vorstehend bereits im Zusammenhang mit einem Beispiel erwähnt wurde, handelt es sich bei der zweiten Verbandlage um einen offenzelligen Polymerschaum, welcher auf seiner ersten Oberfläche eine Vielzahl zur ersten Oberfläche hin offener Hohlräume sowie eine Vielzahl von Stegen aufweist, so dass die auf der Oberfläche der ersten Verbandlage vorhandenen Strukturen haftend mit den auf der Oberfläche der zweiten Verbandlage vorhandenen Strukturen interagieren können. Bei den auf der Oberfläche der zweiten Verbandlage vorhandenen Strukturen handelt sich gemäß dieser Ausführungsform also beispielsweise um Hohlräume oder um die Stege des Schaums.

Gemäß einer hier ganz besonders bevorzugten Ausführungsform umfasst die Vorrichtung als erste Verbandlage eine flexible, perforierte Folie, welche 3-D-Mikrostrukturen aufweist sowie als zweite Verbandlage einen offenzelligen Polymerschaum. Die Folie weist eine erste und eine zweite Seite als Organschutzschicht auf, wobei die erste Seite zum Aufbringen auf den Wundgrund vorgesehen ist. Die 3-D-Mikrostrukturen ragen hierbei ausschließlich aus der zweiten Oberfläche heraus, so dass die zweite Oberfläche eine raue Beschaffenheit aufweist, während die erste Oberfläche eine weitestgehend glatte Beschaffenheit aufweist. Weiter umfasst die Vorrichtung gemäß dieser Ausführungsform einen offenzelligen Polymerschaum mit einer ersten und einer zweiten Seite, wobei die erste Seite zum Aufbringen auf die zweite Seite der Organschutzschicht vorgesehen ist. Der Polymerschaum, bei dem es sich vorzugsweise um einen offenzelligen Schaum aus Polyurethan, insbesondere aus Polyester-Polyurethan handelt, weist auf seiner ersten Oberfläche zur ersten Oberfläche hin offene Hohlräume auf. Die Vorrichtung umfasst weiterhin Verbindungsmittel. Hierbei handelt es sich um auf der Oberfläche von Organschutzschicht und Polymerschaum vorhandene Mikrostrukturen, welche nach Inkontaktbringen von der Organschutzschicht mit dem Schaum eine haftende Wechselwirkung bewirken. Der Polymerschaum kann so nach dem Aufbringen der Organschutzschicht auf freigelegte innere Organe oder auf das Omentum majus lediglich durch Inkontaktbringen mit der Organschutzschicht haftend verbunden werden kann, so dass ein Verschieben der ersten Verbandlage gegenüber der zweiten Verbandlage während der Therapie weitestgehend vermieden werden kann und/oder das gleichzeitige Entfernen von erster und zweiter Verbandlage erleichtert wird. Im Rahmen der hier vorgestellten Ausführungsform, welche eine Folie und einen Schaum umfasst, erweist es sich hinsichtlich der Haftung als besonders vorteilhaft, wenn die erste und die zweite Verbandlage folgende Merkmale in Kombination aufweisen:
a) Die erste Verbandlage (Organschutzschicht) umfasst eine transparente, dreidimensional perforierte Folie aus Polyethylen. Die Perforationsränder ragen aus der zweiten Seite der Folie trichterförmig hervor. Die Höhe der auf der zweiten Seite vorhandenen dreidimensionalen trichterförmigen Struktur beträgt 250 µm bis 600 µm. Die Dicke der Folie (Materialstärke) beträgt 10 µm bis 100 µm. Die offene Fläche der in der Folie vorhandenen Perforationen beträgt mindestens 19 % und höchstens 23 %, vorzugsweise mindestens 20 % und höchstens 22 % der Flächenerstreckung der Folie. Die Anzahl der in der ersten Folie pro Fläche vorhandenen Öffnungen beträgt mindestens 270 pro cm² und höchstens 290 pro cm². Der Durchmesser der Perforationen, gemessen in der Ebene der Folie, beträgt mindestens 250 µm und höchstens 350 µm. Das Flächengewicht der Folie, gemessen nach EN ISO 2286 - 2, beträgt mindestens 55 g/m² und höchstens 65 g/m².
b) Die zweite Verbandlage umfasst einen offenzelligen Schaum, erhältlich durch Umsetzung einer Mischung, umfassend die Komponenten (i) Polyisocyanat, (ii) Polyesterpolyol, (iii) Treibmittel, und (iv) Katalysator. Die Bruchdehnung des Schaums, gemessen gemäß DIN 53571, beträgt 280 % bis 300 %. Der Schaum weist eine Zellzahl (= Anzahl der Poren entlang einer an der Schaumoberfläche in Maschinenrichtung angelegten Geraden pro laufendem cm) von 8 bis 15 pro cm. Die Zellzahl wird bevorzugt mikroskopisch bestimmt. Der Schaum weist eine Rohdichte, gemessen nach DIN EN ISO 845 (Probekörper mit Abmessungen 100 mm x 100 mm x 50 mm, Konditionierung für 24 h im Normklima (23 °C, 50 % rel. Luftfeuchte, 1013 mbar)), zwischen 25,4 und 26,2 kg/m³ und eine Luftdurchlässigkeit, gemessen nach DIN EN ISO 9237 (20 mm Prüfdicke, 20 cm² Prüffläche, 200 Pa Differenzdruck), von 2620 I/(m² sec) bis 2740 I/(m² sec) auf. Ein im Rahmen dieser Ausführungsform besonders gut geeigneter Schaum ist erhältlich aus einer Mischung, umfassend die Komponenten (i) Polyisocyanat, (ii) Polyesterpolyol, (iii) Treibmittel, und (iv) Katalysator, wobei der Polyesterpolyol bevorzugt erhältlich ist durch Umsetzung einer Dicarbonsäure mit 4 bis 8 Kohlenstoffatomen mit einen Dialkohol mit 2 bis 6 Kohlenstoffatomen und/oder bevorzugt ein gewichtsmittleres Molekulargewicht von 500 bis 4000 g/mol aufweist.

Bei Verwendung einer ersten Verbandlage und einer zweiten Verbandlage mit den unter a) und b) vorstehend genannten Merkmalen kann erreicht werden, dass zum Verschieben der ersten Verbandlage gegen die zweite Verbandlage im nassen Zustand eine statische Gleitreibungskraft Fₛ von mindestens 3 N erforderlich ist und dass zum Verschieben der ersten Verbandlage gegen die zweite Verbandlage im trockenen Zustand eine statische Gleitreibungskraft Fₛ von mindestens 6 N erforderlich ist. Bei Verwendung anderer als der hier vorgeschlagenen Materialien kann sich die Notwendigkeit ergeben, die Gleitreibungskraft durch Variation der unter a) und b) vorstehend genannten Parameter zu optimieren. Derartige Versuche können ausgehend von den hier bereits vorgeschlagenen Parameterspannen ohne größeren Aufwand durchgeführt werden.

Sofern in den einschlägigen Normen nicht anders angegeben, werden im Allgemeinen alle Testmethoden bei 23 °C, 50 % rel. Luftfeuchte und 1013 mbar Druck durchgeführt.

Zur verbesserten Applikation einer zum Aufbringen auf freigelegte innere Organe oder auf das Omentum majus, vorgesehen flexiblen Folie, welche somit als Organschutzschicht fungieren kann, wurde in der bisher unveröffentlichten Europäischen Patentanmeldung EP11007377.2-2124 vorgeschlagen, dass auf der Folie mindestens eine vorwiegend zur Mitte der Wundauflage hin offene Tasche auf der im Gebrauch von der Wunde abgewandten Seite der Wundauflage vorhanden ist. Die mindestens eine Tasche kann das gleichmäßige Aufbringen und Auslegen der Wundauflage auf den Wundgrund erheblich erleichtern und wird deshalb gleichfalls zur Verwendung im Zusammenhang mit den hier beschrieben Vorrichtungen, Erzeugnissen und Verfahren empfohlen.

Im Rahmen der Erfindung wird gleichfalls ein Verbandset beansprucht, umfassend:
i) eine erste Verbandlage mit einer ersten und einer zweiten Seite, als Organschutzschicht, wobei die erste Seite zum Aufbringen auf einen Wundgrund, insbesondere auf freigelegte innere Organe oder auf das Omentum majus vorgesehen ist, und wobei die Fläche der Verbandlage 2500 cm² bis 4000 cm², insbesondere 3200 cm² bis 3500 cm² beträgt, beträgt,
ii) eine separat von der ersten Verbandlage bereit gestellte zweite Verbandlage mit einer ersten und einer zweiten Seite, wobei die erste Seite der zweiten Verbandlage zum Aufbringen auf die zweite Seite der ersten Verbandlage vorgesehen ist, und wobei die Fläche der zweiten Verbandlage 500 cm² bis 2000 cm², insbesondere 900 cm² bis 1000 cm² beträgt,
iii) ein Verbindungsmittel, durch das die zweite Verbandlage nach dem Aufbringen der ersten Lage auf freigelegte innere Organe oder auf das Omentum majus mit der ersten Verbandlage verbunden werden kann, so dass ein Verschieben der ersten Verbandlage gegenüber der zweiten Verbandlage während der Therapie weitestgehend vermieden werden kann und/oder das gleichzeitige Entfernen von erster und zweiter Verbandlage erleichtert wird.
iv) optional eine luftdichte Abdeckfolie zum Verschließen des Wundraums.
Bei dem Verbindungsmittel handelt es sich vorzugsweise um an der zweiten Seite der ersten Verbandlage und/oder an der ersten Seite der zweiten Verbandlage angebrachte Materialstücke, beispielsweise um Folienstreifen oder um Klettelemente. Gemäß einer alternativen, jedoch gleichermaßen bevorzugten Ausführungsform des Verbandsets handelt es sich bei dem Verbindungsmittel um an der zweiten Seite der ersten Verbandlage und/oder an der ersten Seite der zweiten Verbandlage vorhandene Mikrostrukturen, welche bewirken können, dass zum Verschieben der ersten Verbandlage gegen die zweite Verbandlage im nassen Zustand eine statische Gleitreibungskraft Fₛ von mindestens 3 N erforderlich ist und dass zum Verschieben der ersten Verbandlage gegen die zweite Verbandlage im trockenen Zustand eine statische Gleitreibungskraft Fₛ von mindestens 6 N erforderlich ist.

### Anwendungsbeispiel I

Bei der Anwendung der erfindungsgemäßen Vorrichtung zur Unterdrucktherapie großflächiger Wunden im Abdominalbereich wird zunächst als Organschutzschicht eine erste Verbandlage auf den dem Anwender zugänglichen Anteil des Wundgrunds gelegt. Bei der ersten Verbandlage handelt es sich vorzugsweise um eine perforierte Folie, welche dreidimensional ausgestaltete Strukturen an ihrer in der Anwendung von der Wunde weg zeigenden Oberfläche umfasst. Der Rand der ersten Verbandlage wird sodann ca. 1 cm bis 15 cm tief in den von Bauchdecke und Wundgrund gebildeten Zwischenraum eingeschoben. Die Wundauflage bildet somit eine für Wundfluid durchlässige Schutzschicht für die freiliegenden inneren Organe. Auf die Organschutzschicht wird im Bereich zwischen den Wundrändern eine zweite Verbandlage mit einer Dickenerstreckung von 3 cm aufgebracht. Dabei ist es für die Wundheilung sehr förderlich, wenn die zweite Verbandlage dergestalt an die Form der Wunde angepasst wird, dass die Wundränder in einem vollständigen Kontakt mit der zweiten Verbandlage stehen. Die Fläche der zweiten Verbandlage ist somit geringer als die Fläche der ersten Verbandlage. Bei der zweiten Verbandlage handelt es sich insbesondere um einen porösen Polymerschaumstoff, welcher an seiner Oberfläche offene Zellen aufweist, so dass die Schaumstoff-Stege mit den dreidimensional ausgestalteten Strukturen der perforierten Folie (Organschutzschicht) nach dem Inkontaktbringen der Oberflächen von erster und zweiter Verbandlage haftend miteinander in Wechselwirkung treten können.

Zum luftdichten Verschließen des Wundbereichs wird ein luftundurchlässiges Abdeckmaterial über die Wunde gelegt. Die Ränder des Abdeckmaterials werden auf die intakte Haut geklebt. Des Weiteren wird ein Unterschlussanschlussstück angebracht, um eine funktionelle Verbindung des Wundraums mit einer außerhalb des Abdeckmaterials befindlichen Unterdruckquelle, beispielsweise einer Unterdruckpumpe, herzustellen, so dass ein Unterdruck im Wundraum herstellbar ist und Flüssigkeiten aus dem Wundraum absaugbar sind. Das Unterdruckanschlussstück wird vorzugsweise auf die von der Wunde abgewandte Außenseite des Abdeckmaterials aufgeklebt, wobei vor dem Aufkleben in das ansonsten luftundurchlässige Abdeckmaterial eine geeignete Öffnung geschnitten wird. Die Unterdrucktherapie wird eingeleitet, indem das Unterdruckanschlussstück mit einer Unterdruckquelle verbunden wird und ein vorzugsweise konstanter Unterdruck für eine Dauer von einigen Minuten bis zu mehreren Tagen angelegt wird.

Ein bevorzugter Unterdruck ist der Bereich von mindestens 80 mm Hg bis höchstens 250 mm Hg, vorzugsweise 125 mm Hg.

Die haftende Verbindung von erster und zweiter Verbandlage gewährleistet, dass ein Verschieben der ersten Verbandlage gegenüber der zweiten Verbandlage während der Therapie weitestgehend vermieden werden kann.

### Anwendungsbeispiel II

Bei der Anwendung der erfindungsgemäßen Vorrichtung zur Unterdrucktherapie großflächiger Wunden im Abdominalbereich wird zunächst als Organschutzschicht eine erste Verbandlage auf den dem Anwender zugänglichen Anteil des Wundgrunds gelegt. Bei der ersten Verbandlage handelt es sich vorzugsweise um eine perforierte Folie, auf deren in der Anwendung von der Wunde weg zeigenden Oberfläche herstellerseitig 6 Folienstreifen über die Oberfläche verteilt angebracht wurden. Die Folienstreifen weisen jeweils eine Länge von 20 cm und eine Breite von 1 cm auf. Die Folienstreifen sind in ihrer Mitte mittels einer punktförmigen Schweißverbindung unlösbar mit der Organschutzschicht verbunden, so dass die Folienstreifen freie Enden mit jeweils ca. 10 cm Länge aufweisen. Der Rand der ersten Verbandlage wird sodann ca. 1 cm bis 15 cm tief in den von Bauchdecke und Wundgrund gebildeten Zwischenraum eingeschoben. Die Wundauflage bildet somit eine für Wundfluid durchlässige Schutzschicht für die freiliegenden inneren Organe. Auf die Organschutzschicht wird im Bereich zwischen den Wundrändern eine zweite Verbandlage mit einer Dickenerstreckung von 5 cm aufgebracht. Bei der zweiten Verbandlage handelt es sich insbesondere um einen porösen Polymerschaumstoff, welcher über seine Oberfläche verteilt durch die gesamte Dickenerstreckung des Schaumstoffs hindurchgehende Schlitze aufweist. Die Schlitze weisen eine Länge von jeweils 3 cm auf. Der Schaumstoff weist pro 100 cm² Fläche 5 Schlitze auf. Zwei bis fünf freie Enden der an der ersten Verbandlage vorhandenen Folienstreifen werden durch die Schlitze der zweiten Schaumstofflage hindurchgeführt und an der von der Wunde weg zeigenden Oberfläche miteinander verknotet, so dass eine feste Verbindung von erster und zweiter Verbandlage während der Unterdruckbehandlung gewährleistet ist. Dabei ist es für die Wundheilung sehr förderlich, wenn die zweite Verbandlage dergestalt an die Form der Wunde angepasst wird, dass die Wundränder in einem vollständigen Kontakt mit der zweiten Verbandlage stehen. Die Fläche der zweiten Verbandlage ist somit geringer als die Fläche der ersten Verbandlage.

Zum luftdichten Verschließen des Wundbereichs wird ein luftundurchlässiges Abdeckmaterial über die Wunde gelegt. Die Ränder des Abdeckmaterials werden auf die intakte Haut geklebt. Des Weiteren wird ein Unterschlussanschlussstück angebracht, um eine funktionelle Verbindung des Wundraums mit einer außerhalb des Abdeckmaterials befindlichen Unterdruckquelle, beispielsweise einer Unterdruckpumpe, herzustellen, so dass ein Unterdruck im Wundraum herstellbar ist und Flüssigkeiten aus dem Wundraum absaugbar sind. Das Unterdruckanschlussstück wird vorzugsweise auf die von der Wunde abgewandte Außenseite des Abdeckmaterials aufgeklebt, wobei vor dem Aufkleben in das ansonsten luftundurchlässige Abdeckmaterial eine geeignete Öffnung geschnitten wird. Die Unterdrucktherapie wird wie in Anwendungsbeispiel I dargestellt durchgeführt.

Die durch die Folienstreifen bewirkte Verbindung von erster und zweiter Verbandlage gewährleistet, dass ein Verschieben der ersten Verbandlage gegenüber der zweiten Verbandlage während der Therapie weitestgehend vermieden werden kann.

Beim Verbandwechsel können die erste und die zweite Verbandlage in einem Arbeitsschritt entfernt werden, da die erste und die zweite Verbandlage fest miteinander verbunden sind.

### Bestimmung der Gleitreibungskraft Fₛ

Die Bestimmung der statischen Gleitreibungskraft Fₛ einer Folien-Oberfläche gegen eine Schaumstoff-Oberfläche erfolgte analog DIN EN ISO 8295 (Ausgabe Oktober 2004) unter Verwendung des in der Norm beschriebenen Prüfgerätes (Zugprüfmaschine der Firma Zwick, Deutschland), wobei ein waagrechter Prüftisch eingesetzt wurde. Es wurden jeweils drei Proben gemessen und der Mittelwert aus den drei Messungen berechnet. Zur Messung der Proben im trockenen Zustand erfolgte vor der Prüfung eine Konditionierung für mindestens 16 h im Normklima bei 23°C, 50 % relative Luftfeuchte, 1013 mbar. Zur Messung der Proben im nassen Zustand wurden die Proben (Schaumstoff und Folie) vollständig im Wasser eingetaucht. Danach wurden die Proben zum Abtropfen 30 s vertikal gehalten und in das Prüfgerät eingespannt. Die Schaumstofflage (Abmessung 150 x 300 mm) wurde an dem Prüftisch befestigt, während die Folienlage (Abmessung 65 x 200 mm) in den Schlitten eingespannt wurde. Die Zeitspanne zwischen dem Abtropfen und dem Beginn der Messung betrug maximal 2 min. Die mit der Folie bespannte quadratische Kontaktgrundfläche des Schlittens betrug 40 cm². Der Schlitten wurde über den unbeweglichen Prüftisch gezogen. Die Prüfgeschwindigkeit betrug 100 mm/min. Das Gewicht des Zugschlittens einschließlich des Reibklotzes betrug 200 g. Die Vorspannung zwischen Schlitten und Kraftmessgerät betrug 0,2 N.

### Beispiel: Bestimmung der Gleitreibungskraft einer ersten Verbandlage gegen eine zweite Verbandlage an einem Prüfgerät

Die Bestimmung der statischen Gleitreibungskraft Fₛ wurde wie vorstehend beschrieben analog DIN EN ISO 8295 durchgeführt.

**Probenpaar 1** - Offenzelliger Schaumstoff gegen eine Schlitzfolie, welche eine weitgehend glatte Oberfläche aufweist.

Schaumstoff: Hydrophober Polyester-Polyurethan-Schaumstoff. Rohdichte gemäß ISO 845 25,8 kg/m³, Stauchhärte gemäß DIN EN ISO 3386-1 3,9 kPa, Zugfestigkeit gemäß DIN 53571 A 170 kPa, Bruchdehnung gemäß DIN 53571 A 290 %, Zellzahl (mikroskopisch bestimmt an einer an der Oberfläche des Schaumes angelegten geraden Linie) 11/cm, Luftdurchlässigkeit gemäß DIN EN ISO 9237 2680I/m²s. Ein derartiger, als Wundauflage verwendbarer Schaumstoff, ist unter der Bezeichnung VivanoMed^{®}-Foam kommerziell erhältlich (Paul Hartmann AG, Deutschland).

**Folie:** Glatte, transparente Polyurethan-Folie mit Schlitzen. In die Folie wurden pro 100 cm² Folienoberfläche ca. 20 über die Fläche der Folie verteilte Schlitze mit einer Länge von jeweils 4,5 mm eingebracht. Die Ausrichtung der Schlitze erfolgte parallel zur Maschinenrichtung (MD) der Folie. Bei einem Einsatz einer derartigen Folie als Wundkontaktschicht beziehungsweise als Organschutzschicht dienen die Schlitze der Durchleitung von Wundexsudat. Die Verwendung geschlitzter Folien als WundkontaktSchicht, insbesondere im Zusammenhang mit der Unterdruck-Therapie, ist aus dem Stand der Technik bekannt.

Die Folie wurde derart in den Messschlitten eingespannt, dass die Bewegung des Schlittens entlang der Maschinenrichtung der Folie erfolgte.

**Probenpaar 2 -** Offenzelliger Schaumstoff gegen eine Schlitzfolie, welche auf einer Oberfläche eine dreidimensional ausgestaltete kraterförmige Struktur umfasst.

**Schaumstoff:** Hydrophober Polyester-Polyurethan-Schaumstoff. Rohdichte gemäß ISO 845 25,8 kg/m³, Stauchhärte gemäß DIN EN ISO 3386-1 3,9 kPa, Zugfestigkeit gemäß DIN 53571 A 170 kPa, Bruchdehnung gemäß DIN 53571A 290 %, Zellzahl (mikroskopisch bestimmt an einer an der Oberfläche des Schaumes angelegten geraden Linie) 11/cm, Luftdurchlässigkeit gemäß DIN EN ISO 9237 2680I/m²s. Ein derartiger, als Wundauflage verwendbarer Schaumstoff, ist unter der Bezeichnung VivanoMed^{®} kommerziell erhältlich (Paul Hartmann AG, Deutschland).

**Folie:** Einseitig raue transparente Polyethylen-Folie mit Perforationen. Die Folie umfasst etwa 280 Perforationen pro cm² Folienfläche mit einem Durchmesser von jeweils 0,3 mm.

Die Perforationen wurden derart in die Folie eingebracht, dass die Perforationsränder von der zweiten Oberfläche der Folie abstehen, so dass auf der zweiten Oberfläche eine kraterförmige, dreidimensional ausgestaltete Struktur vorhanden ist. Die erste Oberfläche der Folie hingegen ist weitgehend glatt ausgebildet. Die offene Fläche der Folie beträgt 21 %. Die Folie wurde derart in den Messschlitten eingespannt, dass die Bewegung des Schlittens entlang der Maschinenrichtung (MD) der Folie erfolgte. Die raue Seite der Folie (zweite Oberfläche) wurde zum Prüftisch hin ausgerichtet, so dass die raue Seite mit dem Schaumstoff in Kontakt stand.

Die zwischen den Materiallagen (Probenpaare) vorhandene statische Gleitreibungskraft wurde jeweils bei trockenen und nassen Proben mittels der vorstehend angegebenen Messmethode bestimmt. Im Folgenden sind die Mittelwerte der gemessenen Gleitreibungskraft Fs aus jeweils drei Messungen angegeben.

| | |
|---|---|
| Probenpaar 1 - trocken | 0,98 N |
| Probenpaar 1 - nass | 0,72 N |
| | |
| Probenpaar 2 - trocken | 10,08 N |
| Probenpaar 2 - nass | 5,86 N |

### Bezugszeichen

- 1: Unterdruckquelle
- 2: Kanister für Wundexsudat
- 3: Wundgrund, insbesondere freigelegte innere Organe oder das Omentum majus
- 4: Bauchdecke
- 5: Wundrand
- 6: luftdichter Abdeckfilm zum Versiegeln des Wundraums
- 7: Unterdruck-Anschlussmittel (Port)
- 8: Unterdruckleitung
- 9: Öffnung in Abdeckfilm
- 10, 20: an eine abdominale Wunde angelegte Vorrichtung zur Anwendung in der therapeutischen Behandlung des offenen Abdomens am menschlichen oder tierischen Körper mittels Unterdruck
- 11, 21, 31: perforierte, flexible Folie (Organschutzschicht)
- 12, 22: offenzelliger Polymerschaum
- 13: zur Oberfläche hin offener Hohlraum in dem offenzelligen Polymerschaum
- 14: an oder nahe der ersten Oberfläche des offenzelligen Polymerschaumes vorhandener Schaumstoff-Steg
- 15: Perforation in der flexiblen Folie
- 16: Perforationsrand in der flexiblen Folie. Der Perforationsrand steht von der zweiten Oberfläche der Folie ab, so dass auf der zweiten Oberfläche der Folie eine dreidimensional ausgestaltete Struktur gebildet wird
- 25: an der zweiten Seite der perforierten, flexiblen Folie unlösbar angebrachtes Klettelement
- 26: an der ersten Seite des offenzelligen Polymerschaums unlösbar angebrachtes Klettelement
- 27, 33: Öffnung in der flexiblen Folie
- 30: Organschutzschicht mit Taschen und Folienstreifen
- 31: perforierte Folie
- 35: an der zweiten Seite der ersten Verbandlage angebrachter Folienstreifen
- 38: zur Mitte der Folie hin offene Tasche
- 39: Befestigungspunkt

### Figuren

Nachstehend wird die erfindungsgemäße Wundauflage bzw. Vorrichtung zur Unterdrucktherapie von Wunden anhand schematischer Zeichnungen (nicht maßstabsgetreu) näher erläutert. Die Erfindung soll jedoch nicht auf die in den Zeichnungen oder in der Beschreibung der Zeichnung dargestellten Ausgestaltungen reduziert verstanden werden. Vielmehr umfasst die Erfindung auch Kombinationen der Einzelmerkmale der alternativen Formen.
Fig. 1a zeigt eine bevorzugte Ausführungsform einer an eine abdominale Wunde angelegten Vorrichtung zur Verwendung bei der Unterdrucktherapie des offenen Abdomens im Querschnitt.
Figur 1b zeigt eine Zeichnung nach einer mikroskopischen Aufnahme (REM) eines als zweite Verbandlage geeigneten offenzelligen Polymerschaumes in der Aufsicht auf die erste Seite. Die Figur zeigt somit ein Oberflächendetail (Originalgröße 6 mm x 6 mm) von Lage 12 aus Figur 1a.
Figuren 1c/d zeigen Zeichnungen nach mikroskopischen Aufnahmen (Auflichtmikroskop) einer als erste Verbandlage geeigneten perforierten Folie in der Aufsicht auf die zweite Seite (Figur 1c; Originalgröße ca. 23 mm x 23 mm) und von der Seite (Figur 1d; Originalgröße ca. 9 mm x 9 mm). Die Figuren zeigen somit ein auf der zweiten Seite von Lage 11 aus Figur 1a vorhandenes Oberflächendetail.
Figur 1e zeigt das Oberflächendetail aus Figur 1d in schematischer Darstellung.
Figur 2a zeigt eine weitere bevorzugte Ausführungsform einer an eine abdominale Wunde angelegten Vorrichtung zur Verwendung bei der Unterdrucktherapie des offenen Abdomens im Querschnitt.
Figur 2b zeigt die erste Verbandlage aus Figur 2a in der Aufsicht auf die zweite Seite.
Figur 2c zeigt die zweite Verbandlage aus Figur 2a in der Aufsicht auf die erste Seite.
Figur 3a/b zeigen eine weitere Ausführungsform einer ersten Verbandlage in der Aufsicht auf die zweite Seite (Figur 3a) und im Querschnitt (Figur 3b).

### Ausführliche Beschreibung der Erfindung

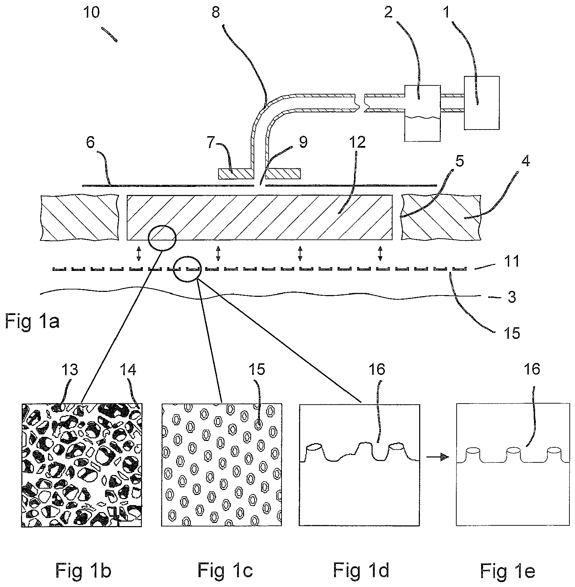

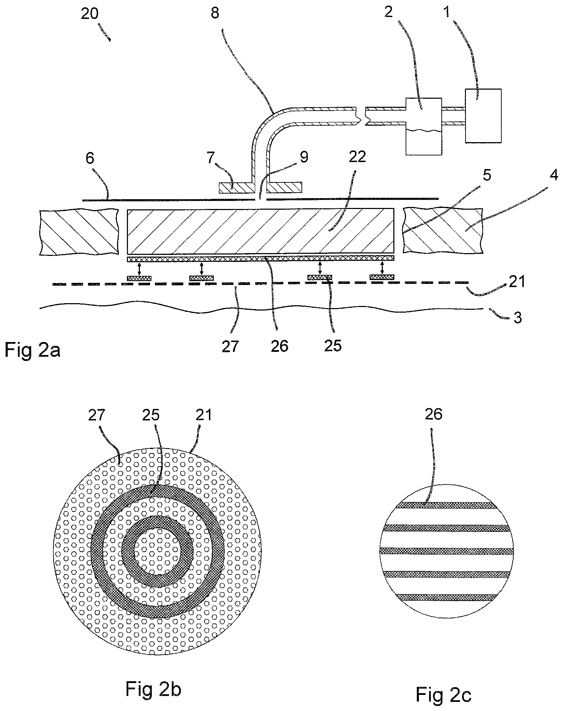

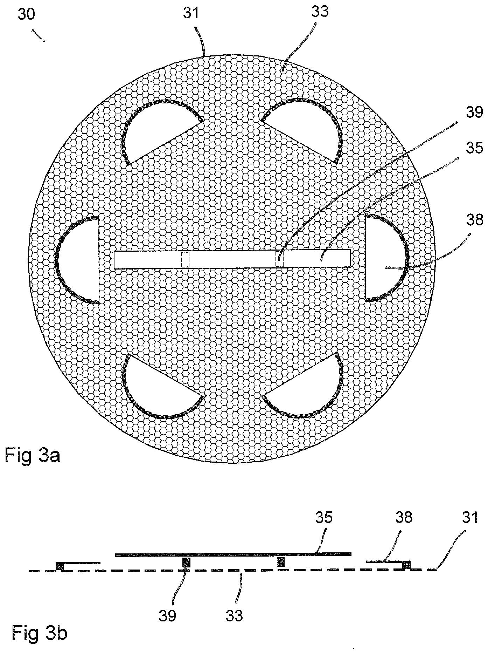

Die Figuren 1 bis 3 stellen beispielhaft bevorzugte Ausführungsformen der Erfindung dar. Den zur Illustration des allgemeinen Erfindungsgedankens ausgewählten Ausführungsformen ist gemeinsam, dass die gezeigten Vorrichtungen (10, 20, 30) das erfindungswesentliche Verbindungsmittel umfassen, mittels dem die zweite Verbandlage (12, 22) nach dem Aufbringen der ersten Lage (11, 21, 31) auf freigelegte innere Organe oder auf das Omentum majus mit der ersten Verbandlage verbunden werden kann, so dass ein Verschieben der ersten Verbandlage (11, 21, 31) gegenüber der zweiten Verbandlage (12, 22) während der Therapie weitestgehend vermieden werden kann und/oder das gleichzeitige Entfernen von erster und zweiter Verbandlage erleichtert wird. Aus den in den Figuren 1 bis 3 dargestellten Beispielen geht hervor, dass das Verbindungsmittel auf unterschiedlichste Weise ausgestaltet werden kann, solange sichergestellt ist, dass die zweite Verbandlage nach dem Aufbringen der ersten Lage auf freigelegte innere Organe oder auf das Omentum majus mit der ersten Verbandlage verbindbar ist, so dass ein Verschieben der ersten Verbandlage (11, 21, 31) gegenüber der zweiten Verbandlage (12, 22) während der Therapie weitestgehend vermieden werden kann und/oder dass eine gleichzeitige Entfernung von erster und zweiter Verbandlage erleichtert wird. Entsprechend ist für den Fachmann offensichtlich, dass eine Vielzahl alternativer Verbindungsmittel in der erfindungsgemäßen Vorrichtung Einsatz finden können. In dem Ausführungsbeispiel nach Figur 1 umfasst das Verbindungsmittel dreidimensional ausgestaltete Strukturen, welche eine Haftung zwischen den Oberflächen von erster und zweiter Verbandlage bewirken. In dem Ausführungsbeispiel nach Figur 2 wird das Verbindungsmittel durch Klettelemente verwirklicht, welche an der ersten und zweiten Verbandlage vorhanden sind. In dem Ausführungsbeispiel nach Figur 3 dagegen ist lediglich auf der ersten Verbandlage ein Folienstreifen vorhanden, welcher vom Anwender mit der zweiten Verbandlage verbunden werden kann. Die hier beschriebenen Ausführungsformen verdeutlichen, dass das Verbindungsmittel beispielsweise nur auf der ersten oder nur auf der zweiten Verbandlage vorhanden sein kann, wie in Fig. 3 gezeigt (das Verbindungsmittel 35, bei dem es sich um einen Folienstreifen handelt, ist lediglich auf der ersten Verbandlage 31 vorhanden). Alternativ können sowohl die erste als auch die zweite Verbandlage das Verbindungsmittel umfassen, wie in den in Figur 1 und Figur 2 dargestellten Beispielen gezeigt.

Figur 1a zeigt in schematischer Darstellung (nicht maßstabsgetreu) eine bevorzugte Ausführungsform der erfindungsgemäßen Vorrichtung an einer abdominalen Wunde. Die Wunde umfasst einen Wundgrund (3) und einen durch die Bauchdecke (4) gebildeten Wundrand (5). Im Falle einer abdominalen Wunde handelt es sich bei dem Wundgrund (3) üblicherweise um freigelegte innere Organe oder um das Omentum majus. Auf den Wundgrund wird als Organschutzschicht zunächst eine flexible, perforierte Folie (11) aufgebracht. Hierbei wird eine erste Seite der Folie (11) direkt in Kontakt mit dem Wundgrund (3) gebracht. Die Ränder der Organschutzschicht werden in den von Wundgrund und Bauchdecke gebildeten Zwischenraum eingeschoben. Auf die Organschutzschicht (11) wird ein an die Größe der Wunde angepasster, retikulierter Polymerschaum (12), beispielsweise ein Polymer Schaum aus Polyurethan, aufgebracht. Der Schaumstoff soll hierbei in direktem Kontakt mit den Wundrändern (5) gebracht werden. Die Dicke der Schaumlage (12) sollte möglichst der Dicke der Bauchdecke (4) entsprechen. Die flexible Folie (11) umfasst eine Vielzahl über die Fläche der Folie verteilter, dreidimensional ausgestalteter Perforationen. Die Folie (11) weist eine glatte erste Seite und eine aufgrund der in die Folie eingebrachten Perforationen (15) aufgeraute zweite Seite auf. Die Figuren 1c und 1d zeigen Zeichnungen nach mikroskopischen Aufnahmen einer für die hier dargestellte Ausführungsform geeigneten perforierten Polyethylen-Folie in der Aufsicht auf die zweite Seite (Figur 1 c) und von der Seite (Figur 1d). Figur 1e zeigt das Oberflächendetail aus Figur 1d in schematischer Darstellung. Aus den Figuren 1c, 1d und 1e ist erkennbar, dass kraterartig aus der Planebene der Folie herausragende Mikrostrukturen (16), welche der zweiten Oberfläche der Folie (11) eine raue Oberflächenbeschaffenheit verleihen, vorhanden sind. Bei den kraterartigen Strukturen (16), welche eine Höhe (senkrecht zur Ebene der Folie gemessene maximale Erstreckung der dreidimensionalen Struktur) von ungefähr 400 µm aufweisen, handelt es sich um Perforationsränder. Im Inneren der Mikrostrukturen (16) sind die Folie durchdringende Öffnungen beziehungsweise Kanäle vorhanden. Der Durchmesser der Öffnungen beträgt jeweils ca. 0,3 mm. Die in Figuren 1a beziehungsweise 1 c gezeigte Folie weist eine offene Fläche von 21 % auf, so dass eine wirksame Ableitung der im Wundbereich während der Therapie abgegebenen Wundexsudate gewährleistet werden kann. Die Figuren 1c und 1d zeigen somit ein Detail der zweiten Oberfläche der in Figur 1a nur schematisch dargestellten flexiblen Folie (11). Figur 1b zeigt eine Zeichnung nach einer mikroskopischen Aufnahme der Oberfläche eines offenzelligen Polymerschaums (12), welcher in der hier gezeigten Ausführungsform in vorteilhafter Weise eine zweite Verbandlage bilden kann. In Figur 1b sind Schaumstoffstege (14) und Hohlräume (13) erkennbar. Die Schaumstoffstege (14) und die Hohlräume (13) bilden an der Schaumstoffoberfläche eine dreidimensional ausgestaltete Mikrostruktur, welche mit den auf der zweiten Seite der flexiblen Folie (11) vorhandenen kraterförmigen Mikrostrukturen (16) eine haftende Verbindung (in Figur 1a wird die zwischen Folie und Schaumstoff auftretende Haftung durch die doppelseitigen Pfeile angedeutet) eingehen können, wenn die zweite Oberfläche der Folie (11) mit der ersten Oberfläche des Schaumstoffs (12) in Kontakt gebracht wird. Bei der in Figur 1a gezeigten Ausführungsform umfasst das Verbindungsmittel somit auf der zweiten Seite der ersten Verbandlage (Folie 11) und auf der ersten Seite der zweiten Verbandlage (Schaumstoff 12) vorhandene Mikrostrukturen (13, 14, 15, 16). Eine unterdruckdichte Abdeckung des Wundgebietes erfolgt durch Aufbringen des luftdichten Abdeckfilms (6), welcher in der Wundumgebung adhäsiv befestigt wird. Normalerweise handelt es sich bei dem zur Abdichtung des Unterdruckverbandes verwendeten Abdeckfilm (6) um einen selbstklebend beschichteten Polyurethanfilm, beispielsweise um das kommerziell erhältliche Produkt Hydrofilm^{®} (Paul Hartmann AG, Deutschland). In den Abdeckfilm (6) wird eine Öffnung (9) mit einem Durchmesser von ungefähr 0,5 cm eingebracht. Ein Unterdruckanschlussmittel (Port, 7), welches mit einer Unterdruckleitung (8) verbunden ist, wird über der Öffnung (9) befestigt, so dass eine Unterdruckkommunikation zwischen dem Lumen der Unterdruckleitung (8) und dem Wundraum hergestellt werden kann. Nach einem Aktivieren der Unterdruckquelle (1) kann Unterdruck im Wundraum hergestellt und Wundsekrete in den Behälter (2) gesaugt werden.

Figur 2a zeigt in schematischer Darstellung (nicht maßstabsgetreu) eine weitere bevorzugte Ausführungsform der erfindungsgemäßen Vorrichtung an einer abdominalen Wunde. Die Vorrichtung umfasst neben den bereits in Figur 1 dargestellten Komponenten Abdeckfilm (6), Port (7), Unterdruckleitung (8), Kanister (2) und Unterdruckquelle (1) eine Folie (21) mit weitgehend glatter Oberflächenstruktur als Organschutzschicht (erste Verbandlage) und eine auf die Folie (21) aufgebrachte Schaumstofflage (zweite Verbandlage 22), wobei sowohl an der Folie (21) als auch an der Schaumstofflage (22) Klettelemente (25, 26) vorhanden sind, welche die erste Verbandlage (21) mit der zweiten Verbandlage (22) klettend verbinden können (in Figur 2a wird die zwischen Folie und Schaumstoff hergestellte klettende Verbindung durch die doppelseitigen Pfeile angedeutet). Entsprechend umfasst das Verbindungsmittel gemäß der in Figur 2a gezeigten bevorzugten Ausführungsform Klettelemente (25, 26). Die klettende Verbindung von erster Verbandlage (21) und zweiter Verbandlage (22) gewährleistet, dass während der Unterdrucktherapie die Verbandlagen nicht gegeneinander verschoben werden. Zudem wird nach Beendigung der Unterdrucktherapie das gleichzeitige Entfernen von Organschutzschicht (21) und Schaumstofflage (22) erleichtert. Vorzugsweise handelt es sich bei der Folie (21) um eine flexible Polymerfolie, beispielsweise aus Polyethylen. Die Folie (21) weist eine Vielzahl von über die Fläche der Folie verteilten Öffnungen (27) auf, welche eine Durchleitung von Wundexsudat ermöglichen. Auf die Form die Öffnungen (27), beispielsweise kreisförmig oder ellipsenförmig, kommt es hierbei weniger an. In der Praxis hat sich jedoch gezeigt, dass die offene Fläche der Folie (21) möglichst zwischen 19 % und 23 % der Folienoberfläche betragen sollte, um ein Verklebten des Wundgrundes (3) mit der Schaumstofflage (22) zu verhindern und gleichzeitig eine ausreichende Ableitung von Exsudat zu gewährleisten. Die zweite Verbandlage umfasst vorzugsweise einen polymeren Schaumstoff (22), insbesondere einen offenzelligen Polyurethan-Schaumstoff. Die Klettelemente (25, 26) sind herstellerseitig auf der im Gebrauch von der Wunde abgewandten zweiten Seite der Organschutzschicht (21) sowie auf der im Gebrauch der Wunde zugewandten ersten Seite der Schaumstofflage (22) unlösbar angebracht. Die Klettelemente (25, 26) sollten möglichst über die Fläche von Organschutzschicht (21) und Schaumstofflage (22) verteilt vorhanden sein. Gleichzeitig sollten die Klettelemente (25, 26) die Durchleitung von Wundexsudat durch die Organschutzschicht (21) und durch die Schaumstofflage möglichst ungehindert zulassen. In vorteilhafter Weise können die Klettelemente (25, 26) auf der Organschutzschicht (21) und/oder auf der Schaumstofflage (22) in streifenförmiger oder ringförmiger Anordnung angebracht sein. Gemäß der in den Figuren 2b und 2c dargestellten bevorzugten Ausführungsform werden die Klettelemente (25) auf die zweite Seite der Organschutzschicht (21) in einer ringförmigen Anordnung aufgebracht, während die Klettelemente (26) auf der ersten Seite der Schaumstofflage (22) in einer streifenförmigen Anordnung vorhanden sind. Eine derartige Anordnung hat sich als besonders vorteilhaft erwiesen, da eine klettende Verbindung der Verbandlagen unabhängig von der Orientierung der Lagen zueinander hergestellt werden kann. Hierbei ist es genauso möglich, dass die Klettelemente (25) auf der zweiten Seite der Organschutzschicht (21) streifenförmig aufgebracht werden, während die Klettelemente (26) auf der ersten Seite der Schaumstofflage (22) in ringförmiger Anordnung vorliegen. Besonders bevorzugt liegen die Klettelemente auf einer Verbandlage (erste oder zweite Verbandlage) in Form zweier oder mehrerer konzentrisch angeordneter Ringe vor, wie in Figur 2b exemplarisch dargestellt.

Die Figuren 3a und 3b zeigen eine weitere Ausführungsform einer im Zusammenhang mit der vorliegenden Erfindung verwendbaren Organschutzschicht (31). Bei der Organschutzschicht (31) handelt es sich um eine flexible Polymerfolie mit einer weitgehend glatten Oberfläche, welche eine Vielzahl von Öffnungen (33) aufweist, um die Durchleitung von Wundexsudat zu gewährleisten. Auf der im Gebrauch von der Wunde abgewandten zweiten Seite der Organschutzschicht (31) ist mindestens ein Folienstreifen (35) vorhanden, welcher eine Verbindung der Schaumstofflage (in Figuren 3a und 3b nicht dargestellt) mit der Organschutzschicht (31) ermöglicht. Weiterhin sind auf der im Gebrauch von der Wunde abgewandten zweiten Seite der Organschutzschicht (31) Taschen (38) als Applikationshilfe vorhanden. Bei der Anwendung des erfindungsgemäßen Erzeugnisses wird zunächst, wie bereits im Zusammenhang mit Figur 1a dargestellt, die Organschutzschicht (31) auf den Wundgrund aufgebracht, wobei die Ränder der Folie (31) in den von Wundgrund und Bauchdecke gebildeten Zwischenraum eingeschoben werden. Um das gleichmäßige Aufbringen und Auslegen der Organschutzschicht (31) auf den Wundgrund zu erleichtern, sind auf der Organschutzschicht (31) vorwiegend zur Mitte der Folie hin offene Taschen (38) vorhanden. Die Taschen (38) können einen tütenartig ausgebildeten Materialabschnitt umfassen, insbesondere einen tütenartig ausgebildeten Folienabschnitt, welcher am Rand der Tasche (38) unlösbar an der Organschutzschicht (31) befestigt ist. Eine unlösbare Befestigung des eine Tasche (38) bildenden Materialabschnittes an der Oberfläche der Organschutzschicht (31) kann beispielsweise durch Verklebten, Verschweißen oder Verpressen erfolgen. Der Anwender kann ein flaches chirurgisches Instrument, beispielsweise einen Bauch- und Darmspatel, in die Tasche (38) einführen und sodann die durch die Tasche vorübergehend am Spatel gehaltene Wundauflage vorsichtig unter die Bauchdecke einschieben. Nach dem Einschieben des Randbereichs der Folie (31) unter die Bauchdecke wird der Spatel wieder aus der Tasche (38) herausgezogen. Vorteilhafterweise umfasst die Organschutzschicht (31) eine Vielzahl von Taschen, beispielsweise sechs Taschen (38), wie in Figur 3a dargestellt. Auf die auf den Wundgrund aufgebrachte Organschutzschicht (31) wird eine an die Größe der Wunde angepasste Schaumstofflage (in Figuren 3a und 3b nicht dargestellt) aufgebracht. Der Anwender kann nun den mindestens einen an der Organschutzschicht (31) vorhandenen Folienstreifen (35) mit der Schaumstofflage verbinden, so dass ein Verschieben der Organschutzschicht (31) gegenüber der zweiten Verbandlage während der Therapie weitestgehend vermieden wird und/oder das gleichzeitige Entfernen beider Verbandlagen nach der Therapie wesentlich erleichtert wird. Hierzu kann der Folienstreifen (35) mit der Schaumstofflage beispielsweise vernäht werden. Es ist auch möglich, dass der Anwender in die Schaumstofflage kleine Öffnungen einbringt, durch welche ein oder mehrere freie Enden des mindestens einen Folienstreifens (35) hindurchgeführt werden können. Gegebenenfalls können die freien Enden der Folienstreifen (35) auf der von der Wunde wegweisenden Seite der Schaumstofflage miteinander verknotet oder auf andere Weise fixiert werden. Der mindestens eine Folienstreifen kann auf der im Gebrauch von der Wunde abgewandten zweiten Seite der Organschutzschicht (31) beispielsweise mittels Befestigungspunkten (39) befestigt sein, wie in Figur 3b im Querschnitt dargestellt. Bei den Befestigungspunkten (39) kann es sich beispielsweise um Klebepunkte oder um Schweißpunkte handeln. Wesentlich ist bei der hier gezeigten Ausführungsform, dass der mindestens eine Folienstreifen (35) mindestens ein freies Ende aufweist, welches nach dem Aufbringen der Organschutzschicht (31) mit der weiteren Verbandlage, insbesondere mit einer Schaumstofflage, verbunden werden kann.

Gemäß einer weiteren, hier nicht dargestellten Ausführungsform, könnten auch an der zweiten Verbandlage Folienstreifen, Bänder oder Fäden vorhanden sein, welche mit den an der ersten Verbandlage vorhandenen Folienstreifen verbindbar sind, beispielsweise durch Verknoten.

## Patentansprüche

1. Vorrichtung, geeignet zur Verwendung bei der Unterdrucktherapie des offenen Abdomens, umfassend
eine erste Verbandlage (11, 21, 31) mit einer ersten und einer zweiten Seite, als Organschutzschicht, wobei die erste Seite zum Aufbringen auf einen Wundgrund (3), insbesondere auf freigelegte innere Organe oder auf das Omentum majus vorgesehen ist,
eine separat von der ersten Verbandlage bereit gestellte zweite Verbandlage (12, 22) mit einer ersten und einer zweiten Seite, wobei die erste Seite der zweiten Verbandlage (12, 22) zum Aufbringen auf die zweite Seite der ersten Verbandlage vorgesehen ist,
ein Verbindungsmittel, durch das die zweite Verbandlage (12, 22) nach dem Aufbringen der ersten Lage (11, 21, 31) auf freigelegte innere Organe oder auf das Omentum majus mit der ersten Verbandlage (11, 21, 31) verbunden werden kann, so dass ein Verschieben der ersten Verbandlage (11, 21, 31) gegenüber der zweiten Verbandlage (12, 22) während der Therapie weitestgehend vermieden werden kann und/oder das gleichzeitige Entfernen von erster (11, 21, 31) und zweiter Verbandlage (12, 22) erleichtert wird, wobei zum Verschieben der ersten Verbandlage (11, 21, 31) im nassen Zustand gegen die zweite Verbandlage (12, 22) im nassen Zustand eine statische Gleitreibungskraft Fₛ, gemessen nach DIN EN ISO 8235, von mindestens 3 N erforderlich ist und/oder wobei zum Verschieben der ersten Verbandlage (11, 21, 31) im trockenen Zustand gegen die zweite Verbandlage (12, 22) im trockenen Zustand eine statische Gleitreibungskraft Fₛ von mindestens 6 N, gemessen nach DIN EN ISO 8235, erforderlich ist.

2. Vorrichtung nach Anspruch 1, wobei die Fläche der ersten Seite der zweiten Verbandlage (12, 22) mindestens 3 % und höchstens 97 %, insbesondere mindestens 25 % und höchstens 90 % der Fläche der ersten Seite der ersten Verbandlage (11, 21, 31) beträgt.

3. Vorrichtung nach Anspruch 1 oder 2, wobei die erste Verbandlage ein Fluid-durchlässiges textiles Material oder eine Fluid-durchlässige flexible Folie (11, 21, 31) aus einem polymeren Material umfasst und/oder wobei die zweite Verbandlage einen Fluid-durchlässigen offenzelligen Polymerschaum (12, 22), insbesondere einen offenzelligen Schaum aus Polyurethan, einen offenzelligen Schaum aus Silikon oder einen offenzelligen Schaum aus Polyvinylalkohol umfasst.

4. Vorrichtung nach einem der vorangehenden Ansprüche, wobei das Verbindungsmittel die Oberfläche der zweiten Seite der ersten Verbandlage (11) und die Oberfläche der ersten Seite der zweiten Verbandlage (12) umfasst.

5. Vorrichtung nach einem der vorangehenden Ansprüche, wobei die Oberfläche der zweiten Seite der ersten Verbandlage (11) und die Oberfläche der ersten Seite der zweiten Verbandlage (12) dreidimensional ausgestaltete Strukturen (13, 14, 15, 16) umfasst, welche eine Haftung zwischen den beiden Oberflächen bewirken.

6. Vorrichtung nach einem der vorangehenden Ansprüche, wobei die erste Verbandlage eine flexible, perforierte Folie (11) umfasst und wobei die Perforationen (15) derart in die Folie eingebracht werden, dass die Perforationsränder (16) von der zweiten Oberfläche der Folie (11) abstehen, so dass auf der zweiten Oberfläche der Folie dreidimensional ausgestaltete Strukturen, insbesondere kraterförmig geformte Strukturen (16), vorhanden sind.

7. Vorrichtung nach einem der vorangehenden Ansprüche 5 oder 6, wobei die zweite Verbandlage einen offenzelligen Polymerschaum (12) umfasst, welcher an oder nahe der ersten Oberfläche vorhandene Stege (14) umfasst und/oder welcher auf seiner ersten Oberfläche zur Oberfläche hin offene Hohlräume (13) umfasst, wobei die Stege (14) und/oder Hohlräume (13) dreidimensional ausgestaltete Strukturen bilden, so dass die auf der Oberfläche der ersten Verbandlage (11) vorhandenen Strukturen haftend mit den auf der Oberfläche der zweiten Verbandlage (12) vorhandenen Strukturen interagieren können.

8. Vorrichtung nach einem der Ansprüche 1 bis 3, wobei das Verbindungsmittel mindestens eine an der zweiten Seite der ersten Verbandlage (21, 31) unlösbar angebrachte zusätzliche Komponente (25, 35) umfasst.

9. Vorrichtung nach Anspruch 8, wobei das Verbindungsmittel eine an der ersten Seite der zweiten Verbandlage (22) unlösbar angebrachte zusätzliche Komponente (26) umfasst.

10. Vorrichtung nach Anspruch 9, wobei die an der ersten Verbandlage (21) und an der zweiten Verbandlage (22) unlösbar angebrachten Komponente jeweils Klettelemente (25, 26) umfassen, welche die erste Verbandlage (21) mit der zweiten Verbandlage (22) klettend verbinden können.

11. Vorrichtung nach Anspruch 8, wobei die zusätzliche Komponente Folien-Streifen (35) umfasst, welche mit der zweiten Verbandlage (31) verbindbar sind.

12. Vorrichtung nach einem der vorangegangenen Ansprüche, wobei die erste Verbandlage eine flexible Folie (31) umfasst und wobei auf der zweiten Seite der Folie (31) mindestens eine vorwiegend zur Mitte der Folie hin offene Tasche (38) vorhanden ist, welche das gleichmäßige Aufbringen und Auslegen der Folie auf den Wundgrund, insbesondere auf die Organe oder auf das Omentum majus erleichtert.

13. Verbandset, geeignet zur Verwendung bei der Unterdrucktherapie des offenen Abdomens, umfassend eine Vorrichtung nach Anspruch 1, wobei die erste Verbandlage (11, 21, 31) eine Fläche von 2500 cm² bis 4000 cm² aufweist und wobei die zweite Verbandlage (12, 22) eine Fläche von 500 cm² bis 2000 cm² aufweist.

14. Verbandset nach Anspruch 13, weiterhin umfassend eine luftdichte Abdeckfolie (6) zum Verschließen des Wundraums.

## Claims

1. Apparatus suitable for use in the negative-pressure therapy of the open abdomen, comprising
a first bandage ply (11, 21, 31) having a first and a second side, as organ-protecting layer, the first side being intended for application to a wound base (3), more particularly exposed internal organs or the greater omentum,
a second bandage ply (12, 22) provided separately from the first bandage ply and having a first and a second side, the first side of the second bandage ply (12, 22) being intended for application to the second side of the first bandage ply,
a joining means by means of which the second bandage ply (12, 22), after application of the first ply (11, 21, 31) to exposed internal organs or to the greater omentum, can be joined to the first bandage ply (11, 21, 31), making it possible to avoid movement of the first bandage ply (11, 21, 31) with respect to the second bandage ply (12, 22) as far as possible during the therapy and/or facilitating simultaneous removal of first (11, 21, 31) and second bandage ply (12, 22), a static sliding friction force Fₛ, measured in accordance with DIN EN ISO 8235, of at least 3 N being required in order to move the first bandage ply (11, 21, 31) in the wet state against the second bandage ply (12, 22) in the wet state and/or a static sliding friction force Fₛ of at least 6 N, measured in accordance with DIN EN ISO 8235, being required in order to move the first bandage ply (11, 21, 31) in the dry state against the second bandage ply (12, 22) in the dry state.

2. Apparatus according to Claim 1, wherein the surface area of the first side of the second bandage ply (12, 22) is at least 3% and not more than 97%, more particularly at least 25% and not more than 90%, of the surface area of the first side of the first bandage ply (11, 21, 31).

3. Apparatus according to Claim 1 or 2, wherein the first bandage ply comprises a fluid-permeable textile material or a fluid-permeable flexible film (11, 21, 31) composed of a polymeric material and/or wherein the second bandage ply comprises a fluid-permeable open-cell polymer foam (12, 22), more particularly an open-cell foam composed of polyurethane, an open-cell foam composed of silicone or an open-cell foam composed of polyvinyl alcohol.

4. Apparatus according to any of the preceding claims, wherein the joining means comprises the surface of the second side of the first bandage ply (11) and the surface of the first side of the second bandage ply (12).

5. Apparatus according to any of the preceding claims, wherein the surface of the second side of the first bandage ply (11) and the surface of the first side of the second bandage ply (12) comprise three-dimensional structures (13, 14, 15, 16) which bring about adhesion between the two surfaces.

6. Apparatus according to any of the preceding claims, wherein the first bandage ply comprises a flexible, perforated film (11) and wherein the perforations (15) are introduced into the film such that the perforation edges (16) protrude from the second surface of the film (11), and so three-dimensional structures, more particularly crater-shaped structures (16), are present on the second surface of the film.

7. Apparatus according to either of preceding Claims 5 and 6, wherein the second bandage ply comprises an open-cell polymer foam (12) which comprises struts (14) present on or close to the first surface and/or which comprises, on its first surface, cavities (13) open towards the surface, the struts (14) and/or cavities (13) forming three-dimensional structures, and so the structures present on the surface of the first bandage ply (11) can interact in an adhesive manner with the structures present on the surface of the second bandage ply (12).

8. Apparatus according to any of Claims 1 to 3, wherein the joining means comprises at least one additional component (25, 35) which is undetachably attached to the second side of the first bandage ply (21, 31).

9. Apparatus according to Claim 8, wherein the joining means comprises an additional component (26) which is undetachably attached to the first side of the second bandage ply (22).

10. Apparatus according to Claim 9, wherein the components undetachably attached to the first bandage ply (21) and to the second bandage ply (22) both comprise hook-and-loop elements (25, 26) which can join the first bandage ply (21) to the second bandage ply (22) by means of hook and loop.

11. Apparatus according to Claim 8, wherein the additional component comprises film strips (35) joinable to the second bandage ply (31).

12. Apparatus according to any of the preceding claims, wherein the first bandage ply comprises a flexible film (31) and wherein, on the second side of the film (31), there is at least one pocket (38) which is predominantly open towards the centre of the film and which facilitates uniform application and laying out of the film on the wound base, more particularly the organs or the greater omentum.

13. Bandage set suitable for use in the negative-pressure therapy of the open abdomen, comprising an apparatus according to Claim 1, the first bandage ply (11, 21, 31) having a surface area of from 2500 cm² to 4000 cm² and the second bandage ply (12, 22) having a surface area of from 500 cm² to 2000 cm².

14. Bandage set according to Claim 13, further comprising an airtight covering film (6) for closing the wound space.

## Revendications

1. Dispositif, utilisable pour le traitement sous vide de l'abdomen ouvert, comprenant
une première couche de pansement (11, 21, 31) avec une première et une deuxième face en tant que couche de protection d'organe, la première face étant prévue pour être appliquée sur un fond de plaie (3), en particulier sur des organes internes exposés ou sur le grand épiploon,
une deuxième couche de pansement (12, 22) prédisposée séparément de la première couche de pansement avec une première et une deuxième face, la première face de la deuxième couche de pansement (12, 22) étant prévue pour être appliquée sur la deuxième face de la première couche de pansement,
un moyen d'assemblage par le biais duquel la deuxième couche de pansement (12, 22), après l'application de la première couche de pansement (11, 21, 31) sur des organes internes exposés ou sur le grand épiploon, peut être raccordée à la première couche de pansement (11, 21, 31) de telle sorte qu'un déplacement de la première couche de pansement (11, 21, 31) par rapport à la deuxième couche de pansement (12, 22) pendant la thérapie peut être empêché au maximum et/ou l'enlèvement simultané de la première couche de pansement (11, 21, 31) et de la deuxième couche de pansement (12, 22) peut être facilité, une force de friction de glissement Fₛ statique, mesurée selon DIN EN ISO 8235, d'au moins 3 N étant nécessaire pour le déplacement de la première couche de pansement (11, 21, 31) à l'état humide contre la deuxième couche de pansement (12, 22) à l'état humide, et/ou une force de friction de glissement Fₛ statique d'au moins 6 N mesurée selon DIN EN ISO 8235 étant nécessaire pour le déplacement de la première couche de pansement (11, 21, 31) à l'état sec contre la deuxième couche de pansement (12, 22) à l'état sec.

2. Dispositif selon la revendication 1, la surface de la première face de la deuxième couche de pansement (12, 22) représentant au moins 3 % et au plus 97 %, en particulier au moins 25 % et au plus 90 %, de la surface de la première face de la première couche de pansement (11, 21, 31).

3. Dispositif selon la revendication 1 ou 2, la première couche de pansement comprenant un matériau textile perméable aux fluides ou une feuille (11, 21, 31) perméable aux fluides en matériau polymère, et/ou la deuxième couche de pansement comprenant une mousse polymère (12, 22) à cellules ouvertes perméable aux fluides, en particulier une mousse de polyuréthane à cellules ouvertes, une mousse de silicone à cellules ouvertes ou une mousse d'alcool polyvinylique à cellules ouvertes.

4. Dispositif selon l'une des revendications précédentes, le moyen d'assemblage comprenant la surface de la deuxième face de la première couche de pansement (11), et la surface de la première face de la deuxième couche de pansement (12).

5. Dispositif selon l'une des revendications précédentes, la surface de la deuxième face de la première couche de pansement (11) et la surface de la première face de la deuxième couche de pansement (12) comprenant des structures (13, 14, 15, 16) constituées en trois dimensions qui provoquent une adhérence entre les deux surfaces.

6. Dispositif selon l'une des revendications précédentes, la première couche de pansement comprenant une feuille (11) souple perforée, et les perforations (15) étant mises en place dans la feuille de telle sorte que les bords de perforation (16) sont écartés de la deuxième surface de la feuille (11) de telle sorte que sur la deuxième surface de la feuille il existe des structures constituées en trois dimensions, en particulier des structures (16) en forme de cratère.

7. Dispositif selon l'une des revendications précédentes 5 ou 6, la deuxième couche de pansement comprenant une mousse polymère (12) à cellules ouvertes qui comprend des nervures (14) présentes sur ou à proximité de la première surface, et/ou qui, sur sa première surface, comprend des cavités (13) ouvertes vers la surface, les nervures (14) et/ou les cavités (13) formant des structures constituées en trois dimensions de telle sorte que les structures présentes sur la surface de la première couche de pansement (11) peuvent interagir par adhérence avec les structures présentes sur la surface de la deuxième couche de pansement (12).

8. Dispositif selon l'une des revendications 1 à 3, le moyen d'assemblage comprenant au moins un composant (25, 35) supplémentaire appliqué de façon non détachable sur la deuxième face de la première couche de pansement (21, 31).

9. Dispositif selon la revendication 8, le moyen d'assemblage comprenant un composant (26) supplémentaire appliqué de façon non détachable sur la première face de la deuxième couche de pansement (22).

10. Dispositif selon la revendication 9, le composant appliqué de façon non détachable sur la première couche de pansement (21) et sur la deuxième couche de pansement (22) comprenant respectivement des éléments auto-agrippants (25, 26) qui peuvent raccorder de façon auto-agrippante la première couche de pansement (21) à la deuxième couche de pansement (22).

11. Dispositif selon la revendication 8, le composant supplémentaire comprenant des bandes de feuille (35) qui peuvent être raccordées à la deuxième couche de pansement (31).

12. Dispositif selon l'une des revendications précédentes, la première couche de pansement comprenant une feuille (31) flexible, et au moins une poche (38) ouverte principalement vers le milieu de la feuille étant présente sur la deuxième face de la feuille (31) et facilitant l'application et la pose uniformes de la feuille sur le fond de plaie, en particulier sur les organes ou le grand épiploon.

13. Set de pansements, utilisable pour le traitement sous vide de l'abdomen ouvert, comprenant un dispositif selon la revendication 1, la première couche de pansement (11, 21, 31) présentant une surface de 2 500 cm² à 4 000 cm², et la deuxième couche de pansement (12, 22) présentant une surface de 500 cm² à 2 000 cm².

14. Set de pansements selon la revendication 13, comprenant également une feuille de couverture (6) étanche à l'air pour la fermeture de l'espace de plaie.
